# EUROPEAN PATENT APPLICATION

(11) **EP 4 840 498 A2**
(43) Date of publication of application: **18.11.2026**
(21) Application number: 26208614.3
(22) Date of filing: 30.06.2020
(51) Int. Cl.: A61N 1/372

(54) **NEURAL BLOCK BY SUPER-THRESHOLD LOW FREQUENCY ELECTRICAL STIMULATION**

(30) Priority: 03.07.2019 US 201962870230 P
(62) Document number(s) of the earlier application(s) in accordance with Art. 76 EPC: 20834433.3 / 3 993 865
(71) Applicant: University of Pittsburgh- Of the Commonwealth System of Higher Education, Pittsburgh PA 15260 (US)
(72) Inventor: TAI, Changfeng, Wexford, Pennsylvania, 15090 (US)
(74) Representative: Kraus & Lederer PartGmbB

(57) **Abstract**

Provided herein is a method of blocking a nerve or neuron including applying an electrical stimulation to the nerve or neuron, wherein the electrical stimulation is of an intensity that is greater than an excitation threshold of the nerve or neuron for a length of time sufficient to produce a block of nerve conduction or neuron excitation.

## Description

### CROSS-REFERENCE TO RELATED APPLICATIONS

This application claims priority to U.S. Provisional Patent Application No. 62/870,230, filed July 3, 2019, the content of which is incorporated herein by reference in its entirety.

### STATEMENT REGARDING FEDERALLY SPONSORED RESEARCH

This invention was made with government support under DK102427 awarded by the National Institutes of Health. The government has certain rights in the invention.

### BACKGROUND OF THE INVENTION

### Field of the Invention

Provided herein is a method of nerve block and related devices, more specifically a method of blocking nerve conduction or neuron excitation by applying super-threshold electrical pulses to the nerve or neuron, and devices for carrying out such methods.

Also provided herein is a method of treating urological or gastrointestinal disorders and related devices, more specifically a method of controlling micturition or defecation by applying super-threshold electrical pulses to the pudendal nerve or a branch thereof, and devices for carrying out such methods.

### Description of Related Art

Blocking nerve conduction or neuron excitation has a broad clinical application to treat many disorders including chronic pain, obesity, heart failure, bladder dysfunction or spasm after spinal cord injury, etc. However, currently electrical nerve block uses high kilohertz (e.g., 4 kHz or greater) electrical pulses in clinical applications, which requires use of a cuff electrode. Use of such electrodes requires invasive surgery to wrap the nerve or neuron with the cuff, limiting the clinical application of such methods.

Accordingly, there is a need in the art for a method of blocking nerve conduction or preventing neuron excitation to provide relief or otherwise treat a condition.

### SUMMARY OF THE INVENTION

Provided herein is a method of blocking a nerve or neuron in a patient, including: applying an electrical stimulation to the nerve or neuron, wherein the electrical stimulation is of an intensity that is greater than an excitation threshold of the nerve or neuron for a length of time sufficient to produce a block of nerve conduction or neuron excitation.

Also provided herein is a device including: a controller; a pulse generator in communication with the controller; and an electrode, optionally one or more electrode leads, configured to be placed near or in contact with a nerve or neuron, the electrode being in electrical communication with the pulse generator, wherein the device is configured to apply an electrical stimulation to a nerve or neuron of a patient, wherein the electrical stimulation is of an intensity that is greater than an excitation threshold of the nerve or neuron for a length of time sufficient to produce a block of nerve conduction or neuron excitation.

Also provided herein is a device including a controller; a pulse generator in communication with the controller; and one or more skin surface electrodes or electromagnetic coils in electrical communication with the pulse generator, wherein the pulse generator and one or more skin surface electrodes or electromagnetic coils are configured to apply an electrical stimulation to a nerve or neuron of a patient, wherein the electrical stimulation is of an intensity that is greater than an excitation threshold of the nerve or neuron for a length of time sufficient to produce a block of nerve conduction or neuron excitation.

Further embodiments or aspects are set forth in the following numbered clauses:
Clause 1: A method of blocking a nerve or neuron, comprising: applying an electrical stimulation to the nerve or neuron, wherein the electrical stimulation is of an intensity that is equal to or greater than an excitation threshold of the nerve or neuron for a length of time sufficient to produce a block of nerve conduction or neuron excitation.
Clause 2: The method of clause 1, wherein the electrical stimulation is delivered at an intensity that is at least five times the excitation threshold of the nerve or neuron, optionally wherein the intensity is of 0.01 mA to 100 mA and/or 1 mV to 500 V, optionally 0.5 mA to 10 mA.
Clause 3: The method of clause 1 or clause 2, wherein the electrical stimulation is delivered at a frequency of 1 Hz to less than 4 kHz, or 1 Hz to 1.5 kHz, or 1 Hz to 1.3 kHz, optionally from 100 Hz to 1 kHz, optionally from 100 Hz to 500 Hz.
Clause 4: The method of any of clauses 1-3, wherein the electrical stimulation is delivered for a period of from 5 seconds to 14 days, 1 minute to 14 days, optionally from 2 minutes to 2 hours, optionally from 1 minute to 7 days, optionally from 1 minute to 5 days, optionally from 2 minutes to 5 minutes, optionally from 5 seconds to 90 seconds.
Clause 5: The method of any of clauses 1-4 wherein the electrical stimulation results in the block of nerve conduction or neuron excitation for at least 10 seconds following cessation of the electrical stimulation.
Clause 6: The method of any of clauses 1-5, wherein the electrical stimulation comprises biphasic electrical pulses.
Clause 7: The method of clause 6, wherein the biphasic pulses are symmetric between the positive and negative phases of the biphasic pulse.
Clause 8: The method of clause 6, wherein the biphasic pulses are asymmetric between the positive and negative phases of the biphasic pulse.
Clause 9: The method of any of clauses 1-8, wherein the electrical stimulation comprises biphasic electrical pulses that are charge-balanced.
Clause 10: The method of any of clauses 1-9, further comprising, once block of nerve conduction or neuron excitation is achieved, stopping application of the electrical stimulation for a period of at least 10 seconds, optionally at least 1 minute, optionally at least 5 minutes, 10 minutes, 15 minutes, 30 minutes, or 180 minutes, wherein the block of nerve conduction or neuron excitation is maintained during the period and, after the period has concluded, resuming electrical stimulation of the nerve or neuron at the same or different intensity and/or at the same or different frequency to continue or prolong the block of nerve conduction or neuron excitation.
Clause 11: The method of any of clauses 1-10, further comprising, once block of nerve conduction or neuron excitation is achieved, maintaining the block by changing the intensity and/or frequency of the electrical stimulation, optionally by reducing the intensity of the electrical stimulation or increasing the frequency of the electrical stimulation.
Clause 12: The method of any of clauses 1-11, wherein the stimulation comprises biphasic pulses at an intensity of 6-9 mA and a frequency of between 100 Hz and 1 kHz, applied for from 10-60 seconds.
Clause 13: A device comprising: a controller; a pulse generator in communication with the controller; and an electrode and/or cuff, optionally one or more electrode leads, configured to be placed near or in contact with a nerve or neuron, the electrode in electrical communication with the pulse generator, wherein the device is configured to apply an electrical stimulation to the nerve or neuron, wherein the electrical stimulation is of an intensity that is greater than an excitation threshold of the nerve or neuron for a length of time sufficient to produce a block of nerve conduction or neuron excitation.
Clause 14: The device of clause 13, wherein the pulse generator is configured to deliver electrical stimulation through the electrode at an intensity that is at least five times the excitation threshold of the nerve or neuron, optionally wherein the intensity is of 0.01 mA to 50 mA and/or 1 mV to 500 V, optionally 0.5 mA to 10 mA.
Clause 15: The device of clause 13 or clause 14, wherein the pulse generator is configured to deliver electrical stimulation through the electrode at a frequency of 1 Hz to less than 4 kHz, or 1 Hz to 1.3 kHz, or 1 Hz to 1.5 kHz, optionally from 100 Hz to 1 kHz, optionally from 100 Hz to 500 Hz, for a period of from 5 seconds to 14 days, or 1 minute to 14 days, optionally from 2 minutes to 2 hours, optionally from 1 minute to 7 days, optionally from 1 minute to 5 days, optionally from 2 minutes to 5 minutes, optionally from 5 seconds to 90 seconds, wherein the electrical stimulation comprises biphasic, charge-balanced electrical pulses.
Clause 16: The device of any of clauses 13-15, wherein the controller is programmed or configured to, once block of nerve conduction or neuron excitation is achieved, instruct the pulse generator to stop application of the electrical stimulation for a period of at least 10 seconds, optionally at least 1 minute, optionally at least 5 minutes, 10 minutes, 15 minutes, 30 minutes, or 180 minutes, wherein the block of nerve conduction or neuron excitation is maintained during the period and, after the period has concluded, resume electrical stimulation of the nerve or neuron at the same or different intensity and/or at the same or different frequency to continue or prolong the block of nerve conduction or neuron excitation.
Clause 17: The device of any of clauses 13-16, wherein the controller is programmed or configured to, once block of nerve conduction or neuron excitation is achieved, instruct the pulse generator to change the intensity and/or frequency of the electrical stimulation, optionally by reducing the intensity of the electrical stimulation or increasing the frequency of the electrical stimulation.
Clause 18: A device comprising: a controller; a pulse generator in communication with the controller; and one or more skin surface electrodes or electromagnetic coils in electrical communication with the pulse generator, wherein the pulse generator and one or more skin surface electrodes or electromagnetic coils are configured to apply an electrical stimulation to a nerve or neuron, wherein the electrical stimulation is of an intensity that is equal to or greater than an excitation threshold of the nerve or neuron for a length of time sufficient to produce a block of nerve conduction or neuron excitation.
Clause 19: The device of clause 18, wherein the pulse generator is configured to deliver electrical stimulation through the electrode or electromagnetic coil at an intensity that is at least five times the excitation threshold of the nerve or neuron, optionally wherein the intensity is of 0.01 mA to 50 mA and/or 1 mV to 500 V, optionally 0.5 mA to 10 mA.
Clause 20: The device of clause 18 or clause 19, wherein the pulse generator is configured to deliver electrical stimulation through the electrode or electromagnetic coil at a frequency of 1 Hz to less than 4 kHz, or 1 Hz to 1.3 kHz, or 1 Hz to 1.5 kHz, optionally from 100 Hz to 1 kHz, optionally from 100 Hz to 500 Hz, for a period of from 5 seconds to 14 days, optionally from 2 minutes to 2 hours, optionally from 1 minute to 7 days, optionally from 1 minute to 5 days, optionally from 2 minutes to 5 minutes, optionally from 5 seconds to 90 seconds, wherein the electrical stimulation comprises biphasic, charge-balanced electrical pulses.
Clause 21: The device of any of clauses 18-20, wherein the controller is programmed or configured to, once block of nerve conduction or neuron excitation is achieved, instruct the pulse generator to stop application of the electrical stimulation for a period of at least 10 seconds, optionally 1 minute, optionally at least 5 minutes, 10 minutes, 15 minutes, 30 minutes, or 180 minutes, wherein the block of nerve conduction or neuron excitation is maintained during the period and, after the period has concluded, resume electrical stimulation of the nerve or neuron at the same or different intensity and/or at the same or different frequency to continue or prolong the block of nerve conduction or neuron excitation.
Clause 22: The device of any of clauses 18-21, wherein the controller is programmed or configured to, once block of nerve conduction or neuron excitation is achieved, instruct the pulse generator to change the intensity and/or frequency of the electrical stimulation, optionally by reducing the intensity of the electrical stimulation or increasing the frequency of the electrical stimulation.
Clause 23: A method of controlling micturition or defecation in a patient comprising: applying a first electrical stimulation to the left pudendal nerve or branches thereof and a second electrical stimulation to the right pudendal nerve or branches thereof of a patient, wherein the first and second electrical stimulation is of an intensity that is equal to or greater than an excitation threshold of the pudendal nerve for a length of time sufficient to produce a conduction block of both pudendal nerves either during or after ending the first and/or second electrical stimulation, thereby inhibiting contraction of the external urethral sphincter (EUS) and/or external anal sphincter (EAS), and either during or immediately after ending the first and/or second electrical stimulation when the EUS/EAS is relaxed, applying a third electrical stimulation to the left or right pudendal nerve at a location central to the blocked pudendal nerve site to induce bladder or colon/rectal contraction, or either during or immediately after ending the first and/or second electrical stimulation when the EUS/EAS is relaxed, applying a third electrical stimulation to a sacral spinal root (S1, S2, S3, or S4) to induce bladder or colon/rectal contraction, or either during or immediately after ending the first and/or second electrical stimulation when the EUS/EAS is relaxed, applying a third electrical stimulation to the spinal cord by epidural electrodes, or skin surface electrodes or electromagnetic coils to induce bladder or colon/rectal contraction, or either during or immediately after ending the first and/or second electrical stimulation when the EUS/EAS is relaxed, applying a third electrical stimulation to pelvic nerve to induce bladder or colon/rectal contraction, or either during or immediately after ending the first and/or second electrical stimulation and during the EUS/EAS relaxation, manually applying pressure to the abdominal area to produce bladder or colon/rectal pressure.
Clause 24: The method of clause 23, wherein the first and second electrical stimulation for pudendal nerve block is delivered at an intensity that is at least five times the excitation threshold of the pudendal nerve, optionally wherein the intensity is of 0.01 mA to 50 mA and/or 1 mV to 500 V, optionally 0.5 mA to 10 mA.
Clause 25: The method of clause 23 or clause 24, wherein the first and second electrical stimulation for pudendal nerve block is delivered at a frequency of 1 Hz to less than 4 kHz, or 1 Hz to 1.3 Hz, or 1 Hz to 1.5 kHz, optionally from 100 Hz to 1 kHz, optionally from 100 Hz to 500 Hz.
Clause 26: The method of any of clauses 23-25, wherein the first and second electrical stimulation for pudendal nerve block is delivered for a period of from 5 seconds to 60 minutes, 1 minute to 60 minutes, optionally from 10 seconds to 90 seconds, optionally from 30 seconds to 60 seconds, optionally from 1 minute to 3 minutes, optionally from 2 minutes to 20 minutes, optionally from 30 minutes to 60 minutes.
Clause 27: The method of any of clauses 23-26, wherein the first and second electrical stimulation results in the block of pudendal nerve conduction for at least 10 seconds following cessation of the electrical stimulation.
Clause 28: The method of any of clauses 23-27, wherein the first and second electrical stimulation comprises biphasic electrical pulses.
Clause 29: The method of clause 28, wherein the biphasic pulses are symmetric between the positive and negative phases of the biphasic pulse.
Clause 30: The method of clause 28, wherein the biphasic pulses are asymmetric between the positive and negative phases of the biphasic pulse.
Clause 31: The method of any of clauses 23-30, wherein the first, second, and third electrical stimulation comprises biphasic electrical pulses that are charge-balanced.
Clause 32: The method of any of clauses 23-31, further comprising, once block of pudendal nerve conduction is achieved, stopping application of the first and/or second electrical stimulation for a period of at least 10 seconds, optionally at least 1 minute, optionally at least 5 minutes, 10 minutes, 15 minutes, 30 minutes, or 180 minutes, wherein the block of pudendal nerve conduction is maintained during the period and, after the period has concluded, resuming the first and/or second electrical stimulation of the pudendal nerve or branch thereof at the same or different intensity and/or at the same or different frequency to continue or prolong the block of pudendal nerve conduction.
Clause 33: The method of any of clauses 23-32, further comprising, once block of pudendal nerve conduction or excitation is achieved, maintaining the block by changing the intensity and/or frequency of the first and/or second electrical stimulation, optionally by reducing the intensity of the electrical stimulation or increasing the frequency of the electrical stimulation.
Clause 34: The method of any of clauses 23-33, wherein the first and second stimulation comprises biphasic pulses at an intensity of 6-9 mA and a frequency of between 100 Hz and 1 kHz, applied for from 10-60 seconds.
Clause 35: The method of any of clauses 23-34, wherein the third electrical stimulation for inducing bladder or colon/rectal contraction and applied to the pudendal nerve, the sacral (S1, S2, S3, or S4) spinal root, spinal cord, or the pelvic nerve has a frequency between 1 Hz to 100 Hz, optionally between 15 Hz and 50 Hz, or optionally 1 Hz to 3 Hz.
Clause 36: The method of any of clauses 23-35, wherein the third electrical stimulation is delivered at an intensity above the excitation threshold of the nerve stimulated and strong enough to induce bladder or colon/rectal contraction causing bladder or colon/rectal pressure to increase more than 20 cm H₂O, optionally wherein the stimulation intensity is of 0.01 mA to 20 mA and/or 1 mV to 20 V, optionally 0.1 mA to 10 mA.
Clause 37: The method of clause 35 or clause 36, wherein the third stimulation is delivered for a period longer than the period of bladder or colon/rectal contraction, optionally for a period of from 10 seconds to 60 minutes, optionally from 30 seconds to 60 seconds, or from 30 seconds to 2 minutes, or from 30 seconds to 3 minutes, or from 30 minutes to 60 minutes.
Clause 38: The method of any of clauses 35-37, further comprising, once the bladder or colon/rectal contraction has ended, stopping application of the third electrical stimulation for a period of at least 10 seconds, optionally at least 1 minute, optionally at least 5 minutes, 10 minutes, 15 minutes, or 30 minutes, after the period has concluded, resuming the third stimulation at the same or different intensity and/or at the same or different frequency to induce bladder or colon contraction again while the pudendal nerves are blocked and the EUS/EAS is relaxed.
Clause 39: A method of controlling micturition or defecation in a patient comprising: applying first electrical stimulation to either left or right pudendal nerve or branches thereof of a subject, wherein the electrical stimulation is of an intensity that is equal to or greater than an excitation threshold of the pudendal nerve for a length of time sufficient to produce a block of pudendal nerve conduction either during or after ending the electrical stimulation, thereby inhibiting contraction of the external urethral sphincter (EUS) and/or external anal sphincter (EAS), and either during or immediately after ending the first electrical stimulation when the EUS/EAS is relaxed, applying a second electrical stimulation to the same side of the blocked pudendal nerve at a location central to the blocked pudendal nerve site to induce bladder or colon/rectal contraction, or either during or immediately after ending the first electrical stimulation when the EUS/EAS is relaxed, applying a second electrical stimulation to a sacral spinal root (S1, S2, S3, or S4) to induce bladder or colon/rectal contraction, or either during or immediately after ending the first electrical stimulation when the EUS/EAS is relaxed, applying a second electrical stimulation to the spinal cord by epidural electrodes, or skin surface electrodes or electromagnetic coils to induce bladder or colon/rectal contraction, or either during or immediately after ending the first electrical stimulation when the EUS/EAS is relaxed, applying a second electrical stimulation to pelvic nerve to induce bladder or colon/rectal contraction, or either during or immediately after ending the electrical stimulation when the EUS/EAS is relaxed, manually applying pressure to the abdominal area to produce bladder or colon/rectal pressure.
Clause 40: The method of clause 39, wherein the first electrical stimulation for pudendal nerve block is delivered at an intensity that is at least five times the excitation threshold of the pudendal nerve, optionally wherein the intensity is of 0.01 mA to 50 mA and/or 1 mV to 500 V, optionally 0.5 mA to 10 mA.
Clause 41: The method of clause 39 or clause 40, wherein the first electrical stimulation for pudendal nerve block is delivered at a frequency of 1 Hz to less than 4 kHz, or 1 Hz to 1.3 Hz, or 1 Hz to 1.5 kHz, optionally from 100 Hz to 1 kHz, optionally from 100 Hz to 500 Hz.
Clause 42: The method of any of clauses 39-41, wherein the first electrical stimulation for pudendal nerve block is delivered for a period of from 5 seconds to 60 minutes, 1 minute to 60 minutes, optionally from 10 seconds to 90 seconds, optionally from 30 seconds to 60 seconds, optionally from 1 minute to 3 minutes, optionally from 2 minutes to 20 minutes, optionally from 30 minutes to 60 minutes.
Clause 43: The method of any of clauses 39-42, wherein the first electrical stimulation results in the block of pudendal nerve conduction for at least 10 seconds following cessation of the electrical stimulation.
Clause 44: The method of any of clauses 39-43, wherein the first electrical stimulation comprises biphasic electrical pulses.
Clause 45: The method of clause 44, wherein the biphasic pulses are symmetric between the positive and negative phases of the biphasic pulse.
Clause 46: The method of clause 44, wherein the biphasic pulses are asymmetric between the positive and negative phases of the biphasic pulse.
Clause 47: The method of any of clauses 39-46, wherein the first and second electrical stimulation comprises biphasic electrical pulses that are charge-balanced.
Clause 48: The method of any of clauses 39-47, further comprising, once block of pudendal nerve conduction is achieved, stopping application of the first electrical stimulation for a period of at least 10 seconds, optionally at least 1 minute, optionally at least 5 minutes, 10 minutes, 15 minutes, 30 minutes, or 180 minutes, wherein the block of pudendal nerve conduction is maintained during the period and, after the period has concluded, resuming the first electrical stimulation of the pudendal nerve or branch thereof at the same or different intensity and/or at the same or different frequency to continue or prolong the block of pudendal nerve conduction.
Clause 49: The method of any of clauses 39-48, further comprising, once block of pudendal nerve conduction or excitation is achieved, maintaining the block by changing the intensity and/or frequency of the first electrical stimulation, optionally by reducing the intensity of the electrical stimulation or increasing the frequency of the electrical stimulation.
Clause 50: The method of any of clauses 39-49, wherein the first stimulation comprises biphasic pulses at an intensity of 6-9 mA and a frequency of between 100 Hz and 1 kHz, applied for from 10-60 seconds.
Clause 51: The method of any of clauses 39-50, wherein the second electrical stimulation for inducing bladder or colon/rectal contraction and applied to the pudendal nerve, the sacral (S1, S2, S3, or S4) spinal root, spinal cord, or the pelvic nerve has a frequency between 1 Hz to 100 Hz, optionally between 15 Hz and 50 Hz, or optionally between 1 Hz and 3 Hz.
Clause 52: The method of clause 51, wherein the second electrical stimulation is delivered at an intensity above the excitation threshold of the nerve stimulated and strong enough to induce bladder or colon/rectal contraction causing bladder or colon/rectal pressure to increase more than 20 cm H₂O, optionally wherein the stimulation intensity is of 0.01 mA to 20 mA and/or 1 mV to 20 V, optionally 0.1 mA to 10 mA.
Clause 53: The method of clause 51 or clause 52, wherein the second stimulation is delivered for a period longer than the period of bladder or colon/rectal contraction, optionally for a period of from 10 seconds to 60 minutes, optionally from 30 seconds to 60 seconds, or from 30 seconds to 2 minutes, or from 30 seconds to 3 minutes, or from 30 minutes to 60 minutes.
Clause 54: The method of any of clauses 51-53, further comprising, once the bladder or colon/rectal contraction has ended, stopping application of the second electrical stimulation for a period of at least 10 seconds, optionally at least 1 minute, optionally at least 5 minutes, 10 minutes, 15 minutes, or 30 minutes, after the period has concluded, resuming the second stimulation at the same or different intensity and/or at the same or different frequency to induce bladder or colon/rectal contraction again while the pudendal nerves are blocked and the EUS/EAS is relaxed.
Clause 55: A device of controlling micturition or defecation in a patient comprising: a controller; one or two pulse generators in communication with the controller; and one or more lead and/or cuff electrodes, optionally two electrodes, configured to be placed near or in contact with the left and/or right pudendal nerves, the electrodes in electrical communication with the first pulse generator, wherein the device is configured to apply an electrical stimulation to the pudendal nerve, wherein the electrical stimulation is of an intensity that is equal to or greater than an excitation threshold of the pudendal nerve for a length of time sufficient to produce a block of pudendal nerve conduction either during or after ending the electrical stimulation, thereby inhibiting contraction of the external urethral sphincter (EUS) and/or the external anal sphincter (EAS); and another electrode, configured to be placed near or in contact with the blocked pudendal nerve at a site central to the blocked site, or be placed near or in contact with the sacral (S1, S2, S3, or S4) spinal root, spinal cord, or pelvic nerve, the electrode in electrical communication with the first pulse generator, wherein the device is configured to apply electrical stimulation using the electrode either during or immediately after the ending of pudendal nerve blocking stimulation at an intensity that is greater than an excitation threshold of the pudendal nerve, sacral (S1, S2, S3, or S4) spinal root, spinal cord, or pelvic nerve to induce bladder or colon/rectal contraction; or skin surface electrodes or electromagnetic coils, configured to be placed on the skin surface along the patient's spine and in electrical communication with the second pulse generator, and wherein the device is configured to apply electrical stimulation using the skin surface electrodes or electromagnetic coils either during or immediately after the ending of pudendal nerve blocking stimulation at an intensity that is greater than an excitation threshold of the spinal cord to induce bladder or colon/rectal contraction.
Clause 56: The device of clause 55, wherein the electrical stimulation for pudendal nerve block is delivered at an intensity that is at least five times the excitation threshold of the pudendal nerve, optionally wherein the intensity is of 0.01 mA to 50 mA and/or 1 mV to 500 V, optionally 0.5 mA to 10 mA.
Clause 57: The device of clause 55 or clause 56, wherein the electrical stimulation for pudendal nerve block is delivered at a frequency of 1 Hz to less than 4 kHz, or 1 Hz to 1.3 Hz, or 1 Hz to 1.5 kHz, optionally from 100 Hz to 1 kHz, optionally from 100 Hz to 500 Hz.
Clause 58: The device of any of clauses 55-57, wherein the electrical stimulation for pudendal nerve block is delivered for a period of from 5 seconds to 60 minutes, 1 minute to 60 minutes, optionally from 10 seconds to 90 seconds, optionally from 30 seconds to 60 seconds, optionally from 1 minute to 5 minutes, optionally from 2 minutes to 20 minutes, optionally from 30 minutes to 60 minutes.
Clause 59: The device of any of clauses 55-58, wherein the electrical stimulation results in the block of pudendal nerve conduction for at least 10 seconds following cessation of the electrical stimulation.
Clause 60: The device of any of clauses 55-59, wherein the electrical stimulation for pudendal nerve block comprises biphasic electrical pulses.
Clause 61: The device of clause 60, wherein the biphasic pulses are symmetric between the positive and negative phases of the biphasic pulse.
Clause 62: The device of clause 60, wherein the biphasic pulses are asymmetric between the positive and negative phases of the biphasic pulse.
Clause 63: The device of any of clauses 55-62, wherein the electrical stimulation comprises biphasic electrical pulses that are charge-balanced.
Clause 64: The device of any of clauses 55-63, further comprising, once block of pudendal nerve conduction is achieved, stopping application of the pudendal nerve blocking electrical stimulation for a period of at least 10 seconds, optionally at least 1 minute, optionally at least 5 minutes, 10 minutes, 15 minutes, 30 minutes, or 180 minutes, wherein the block of pudendal nerve conduction is maintained during the period and, after the period has concluded, resuming the electrical stimulation of the pudendal nerve or branch thereof at the same or different intensity and/or at the same or different frequency to continue or prolong the block of pudendal nerve conduction.
Clause 65: The device of any of clauses 55-63, further comprising, once block of pudendal nerve conduction is achieved, maintaining the block by changing the intensity and/or frequency of the pudendal nerve blocking electrical stimulation, optionally by reducing the intensity of the electrical stimulation or increasing the frequency of the electrical stimulation.
Clause 66: The device of any of clauses 55-65, wherein the electrical stimulation for inducing bladder or colon/rectal contraction and applied to the pudendal nerve, the sacral (S1, S2, S3, or S4) spinal root, spinal cord, or the pelvic nerve has a frequency between 1 Hz to 100 Hz, optionally between 15 Hz and 50 Hz, or optionally between 1 Hz to 3 Hz.
Clause 67: The device of clause 66, wherein the stimulation is delivered at an intensity above the excitation threshold of the nerve stimulated and strong enough to induce bladder contraction causing bladder or colon/rectal pressure to increase more than 20 cm H₂O, optionally wherein the intensity is of 0.01 mA to 20 mA and/or 1 mV to 20 V, optionally 0.1 mA to 10 mA.
Clause 68: The device of clause 66 or clause 67, wherein the stimulation is delivered for a period longer than the period of bladder or colon/rectal contraction, optionally for a period of from 10 seconds to 60 minutes, optionally from 30 seconds to 60 seconds, or from 30 seconds to 2 minutes, or from 30 seconds to 3 minutes, or from 30 minutes to 60 minutes.
Clause 69: The device of any of clauses 66-68, further comprising, once the bladder or colon/rectal contraction has ended, stopping application of the electrical stimulation for a period of at least 10 seconds, optionally at least 1 minute, optionally at least 5 minutes, 10 minutes, 15 minutes, 30 minutes, or 180 minutes, after the period has concluded, resuming the stimulation at the same or different intensity and/or at the same or different frequency to induce bladder or colon/rectal contraction again while the pudendal nerves are blocked and the EUS/EAS is relaxed.
Clause 70: A device of controlling micturition or defecation in a patient comprising: a controller; a pulse generator in communication with the controller; and one or more electrode leads and/or cuffs, optionally two electrodes, configured to be placed near or in contact with the left and/or right pudendal nerves, the electrodes in electrical communication with the pulse generator, wherein the device is configured to apply an electrical stimulation to the pudendal nerve, wherein the electrical stimulation is of an intensity that is equal to or greater than an excitation threshold of the pudendal nerve for a length of time sufficient to produce a block of pudendal nerve conduction either during or after ending the electrical stimulation, thereby inhibiting contraction of the external urethral sphincter (EUS) and/or the external anal sphincter (EAS).
Clause 71: The device of clause 70, wherein the electrical stimulation for pudendal nerve block is delivered at an intensity that is at least five times the excitation threshold of the pudendal nerve, optionally wherein the intensity is of 0.01 mA to 50 mA and/or 1 mV to 500 V, optionally 0.5 mA to 10 mA.
Clause 72: The device of clause 70 or clause 71, wherein the electrical stimulation for pudendal nerve block is delivered at a frequency of 1 Hz to less than 4 kHz, or 1 Hz to 1.3 Hz, or 1 Hz to 1.5 kHz, optionally from 100 Hz to 1 kHz, optionally from 100 Hz to 500 Hz.
Clause 73: The device of any of clauses 70-72, wherein the electrical stimulation for pudendal nerve block is delivered for a period of from 5 seconds to 60 minutes, 1 minute to 60 minutes, optionally from 10 seconds to 90 seconds, optionally from 30 seconds to 60 seconds, optionally from 1 minute to 5 minutes, optionally from 2 minutes to 20 minutes, optionally from 30 minutes to 60 minutes.
Clause 74: The device of any of clauses 70-73, wherein the electrical stimulation results in the block of pudendal nerve conduction for at least 10 seconds following cessation of the electrical stimulation.
Clause 75: The device of any of clauses 70-74, wherein the electrical stimulation comprises biphasic electrical pulses.
Clause 76: The method of clause 75, wherein the biphasic pulses are symmetric between the positive and negative phases of the biphasic pulse.
Clause 77: The method of clause 75, wherein the biphasic pulses are asymmetric between the positive and negative phases of the biphasic pulse.
Clause 78: The method of any of clauses 70-77, wherein the electrical stimulation comprises biphasic electrical pulses that are charge-balanced.
Clause 79: The device of any of clauses 70-78, further comprising, once block of pudendal nerve conduction is achieved, stopping application of the electrical stimulation for a period of at least 10 seconds, optionally at least 1 minute, optionally at least 5 minutes, 10 minutes, 15 minutes, 30 minutes, or 180 minutes, wherein the block of pudendal nerve conduction is maintained during the period and, after the period has concluded, resuming electrical stimulation of the pudendal nerve or branch thereof at the same or different intensity and/or at the same or different frequency to continue or prolong the block of pudendal nerve conduction.
Clause 80: The device of any of clauses 70-79, further comprising, once block of pudendal nerve conduction is achieved, maintaining the block by changing the intensity and/or frequency of the electrical stimulation, optionally by reducing the intensity of the electrical stimulation or increasing the frequency of the electrical stimulation.

### BRIEF DESCRIPTION OF THE DRAWINGS

Additional advantages and details of the methods and devices are explained in greater detail below with reference to the exemplary embodiments and aspects, and the following figures in which:

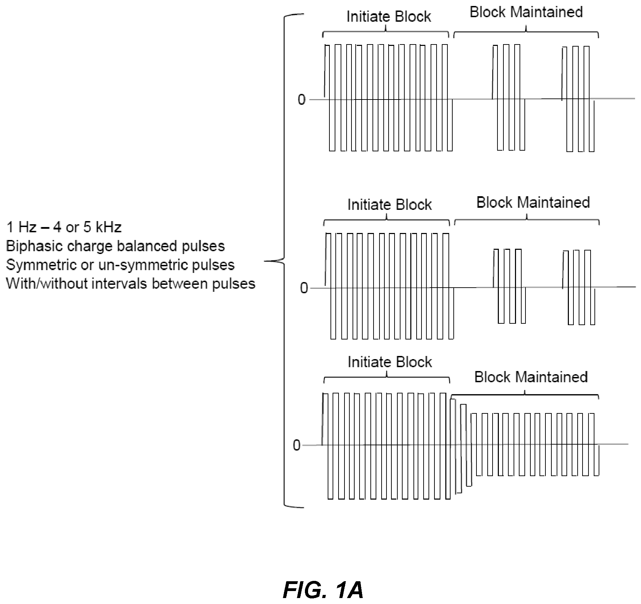

**FIGS. 1A-1B** show that once block is achieved with super-threshold stimulation at low frequency, it can be maintained in a variety of different manners/frequencies;

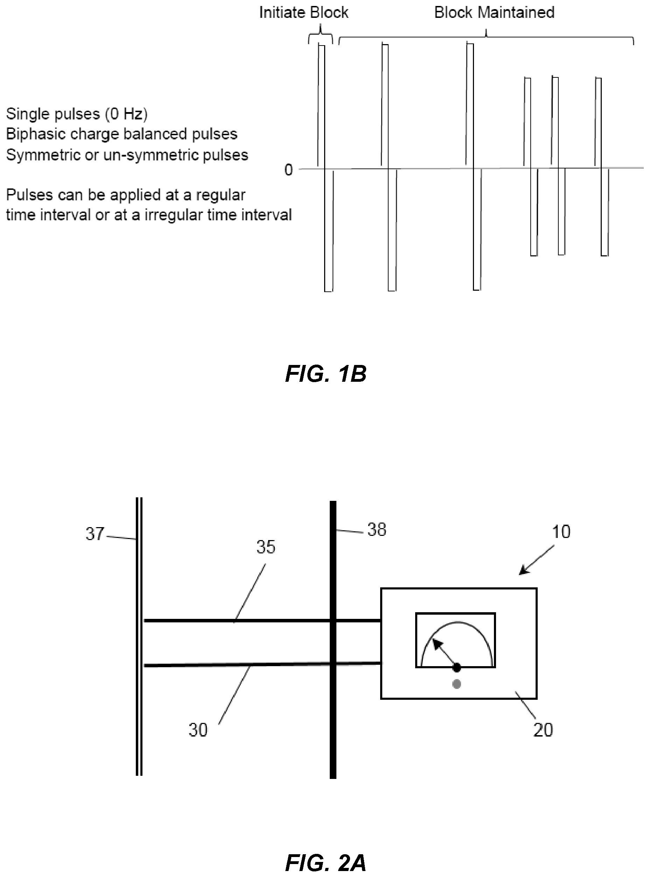

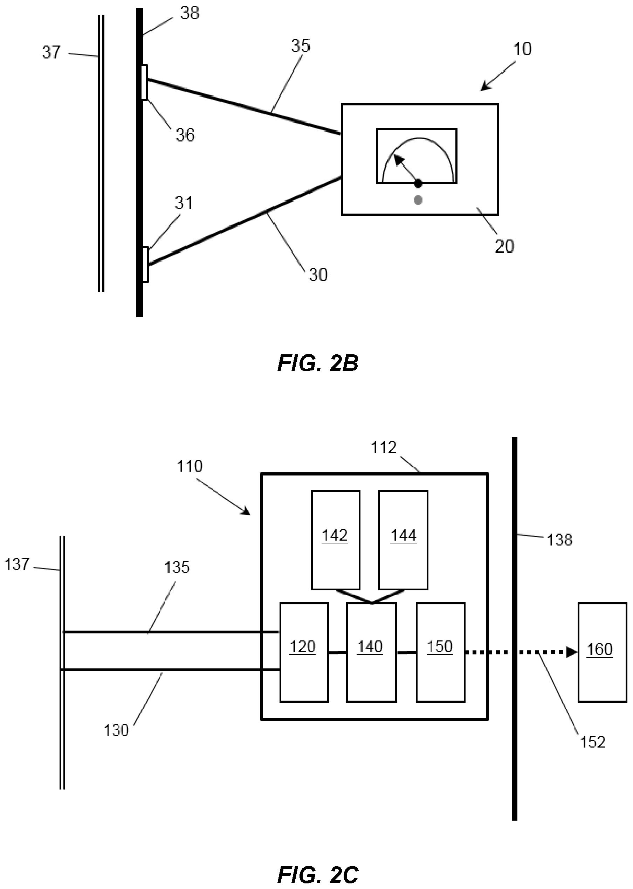

**FIGS. 2A-2C** are schematic diagrams of various external systems (Figures 2A and 2B), and implantable systems (Figure 2C) for use in blocking nerves as described herein;

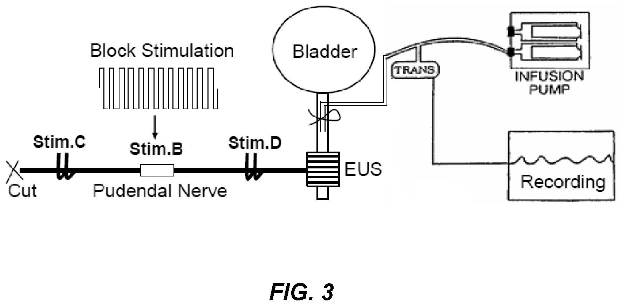

**FIG. 3** shows an experimental setup for application of blocking stimulation. Block stimulation is applied to the pudendal nerve by a tripolar cuff electrode (Stim.B) to block propagation of the action potentials induced by a bipolar hook electrode at a central site (Stim.C). Another bipolar hook electrode is placed at a distal site (Stim.D) to confirm that the external urethral sphincter (EUS) is not fatigued. The urethra is slowly perfused by an infusion pump so that the EUS contraction can be recorded by the increase in urethral pressure;

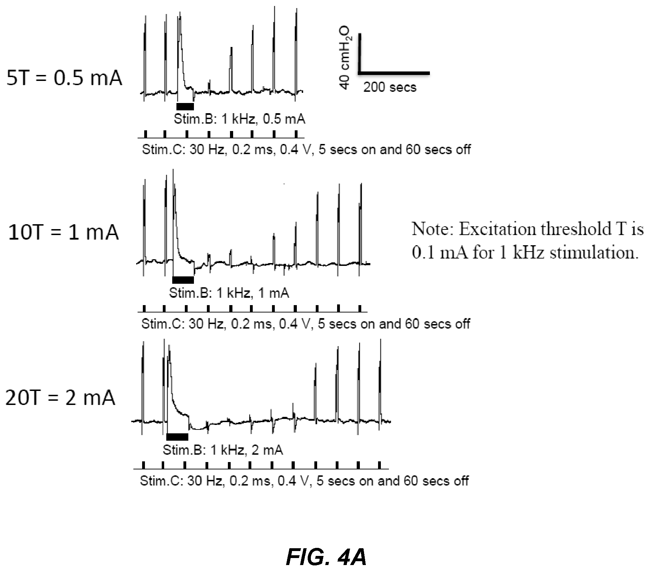

**FIGS. 4A-4B** show effect of different intensities on post-stimulation block delivered at 1 kHz, where excitation threshold T is 0.1 mA at 1 kHz;

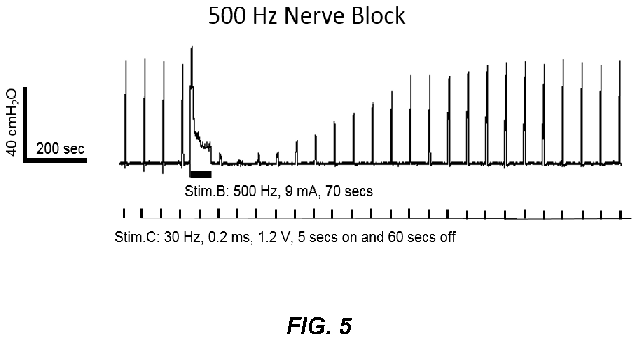

**FIG. 5** shows the post-stimulation blocking effect of stimulation delivered at 500 Hz, intensity of 9 mA, for 70 seconds;

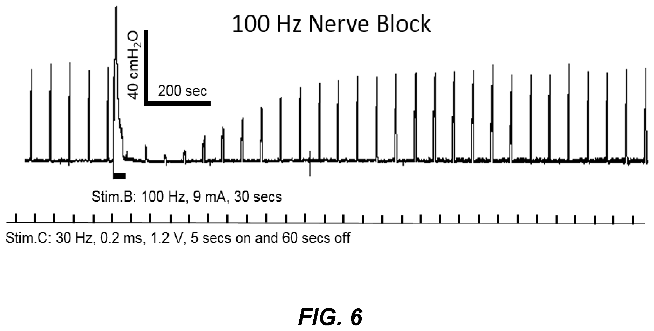

**FIG. 6** shows the post-stimulation blocking effect of stimulation delivered at 100 Hz, intensity of 9 mA, for 30 seconds;

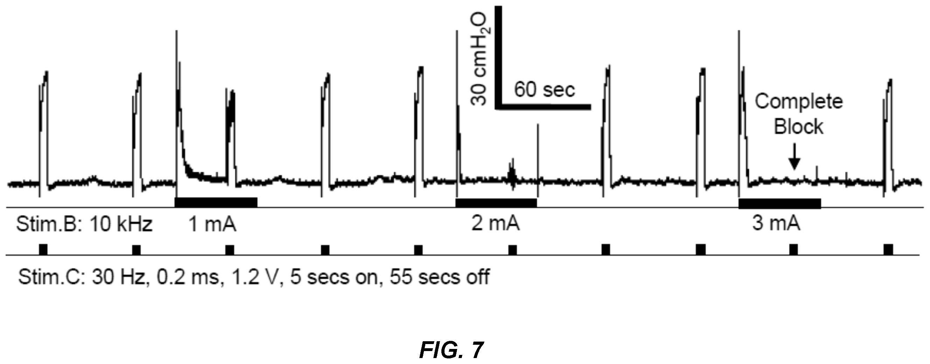

**FIG. 7** shows determination of the block threshold (T) as the stimulus intensity at which 10 kHz stimulation (Stim.B) completely blocked the EUS contraction induced by the 30 Hz intermittent (5 secs on, 55 secs off) stimulation at the central site (Stim.C). T = 3 mA as shown in this figure. The thick black bars under the pressure trace indicate the durations of stimulation. Please note that the 10 kHz Stim.B did not induce a post-stimulation block. A much longer stimulation duration (10-30 minutes) was required for 10 kHz to induce a post-stimulation block;

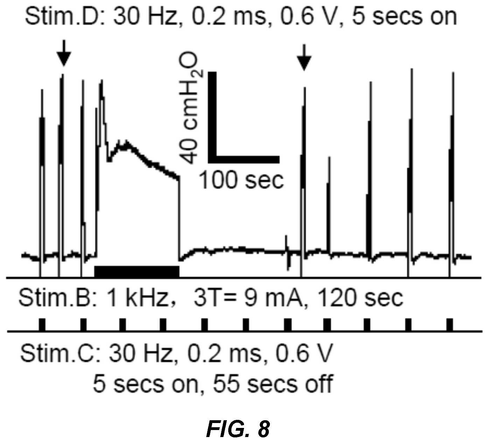

**FIG. 8** shows complete nerve block occurs after the end of the 1 kHz stimulation. The arrows indicate the EUS contractions induced by the 30 Hz stimulation at the distal site (Stim.D). The thick bars under the EUS pressure trace indicate the stimulation durations for Stim.B and Stim.C. After ending the 1 kHz Stim.B, EUS contraction can still be induced by the distal Stim.D but the contractions induced by the central Stim.C are completely blocked initially and then gradually recover, indicating that nerve conduction block occurs locally at Stim.B and the EUS is not fatigued;

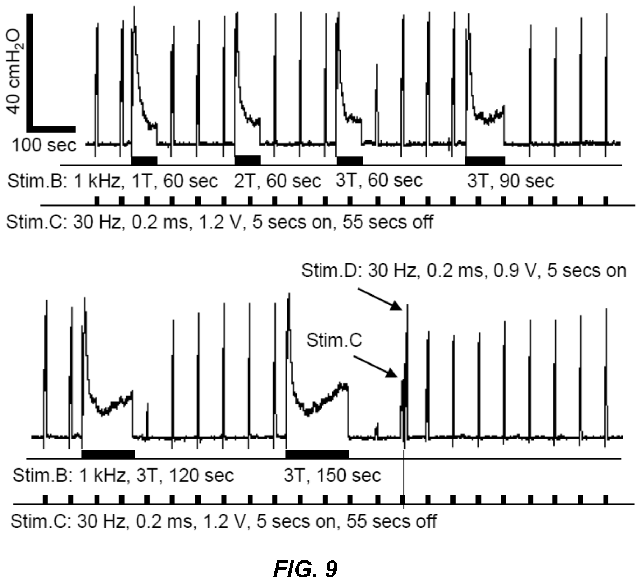

**FIG. 9** shows a protocol to determine the minimal intensity and duration for 1 kHz stimulation to induce a post-stimulation block that can reduce EUS contractions to <5 cm H₂O urethral pressure, i.e., a complete post-stimulation block. The bottom trace is the continuation of the top trace. The 2 arrows indicate the EUS contractions induced by the 30 Hz stimulation at the distal site (Stim.D) and the central site (Stim.C). The thick bars under the EUS pressure trace indicate the stimulation durations for Stim.B and Stim.C. T = 3 mA;

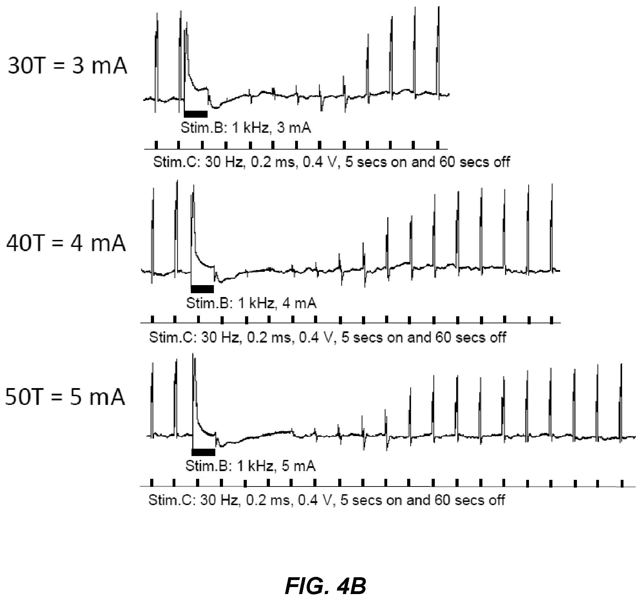

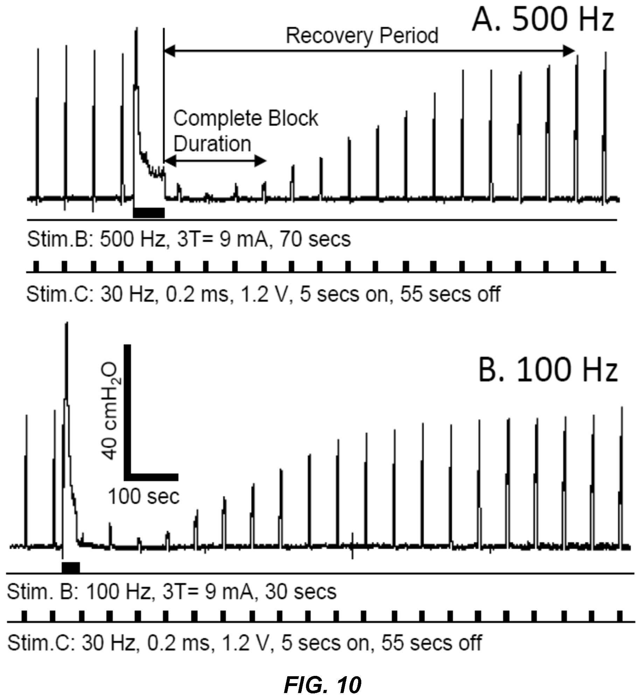

**FIG. 10** shows the minimal stimulation intensity and duration to induce a complete post-stimulation were also determined for 500 Hz (A) or 100 Hz (B) stimulation. The thick bars under the EUS pressure trace indicate the stimulation durations for Stim.B and Stim.C. The data were obtained in the same experiment as shown in FIG. 4. The recovery period is defined as the time period required for the EUS contraction pressure to reach >90% of the pre-stimulation pressure. The complete block duration is defined as the post-stimulation period during which Stim.C can only induce <5 cm H₂O urethral pressure;

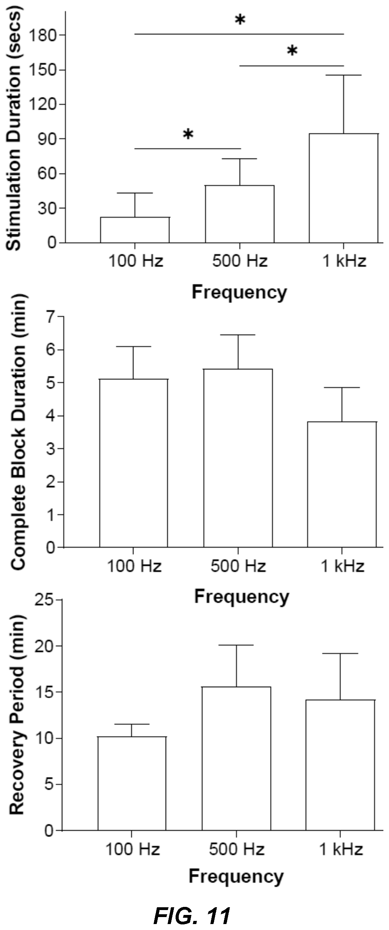

**FIG. 11** shows effects of stimulation frequency on the complete post-low frequency blocking stimulation (LFBS) block induced by LFBS at the minimal intensity and duration. A longer stimulation duration is required for higher frequencies to induce a complete post-LFBS block (A). However, complete block duration (B) and recovery period (C) do not change with stimulation frequency (p>0.05, un-paired t-test). The nerve block can occur at intensities of 1-3 T (3-9 mA) at different frequencies. N = 6 for 100 Hz, N = 9 for 500 Hz, and N = 14 for 1 kHz. * indicates significant different (p<0.05, unpaired t-test); and

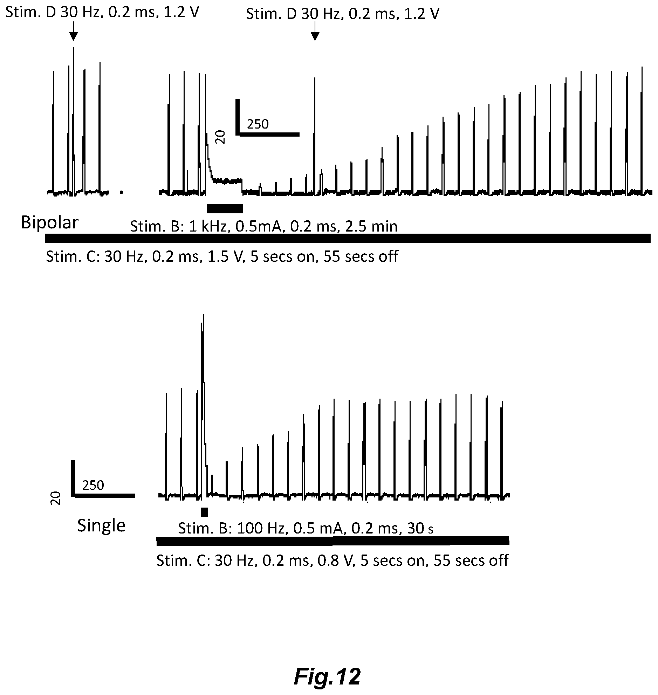

**FIG. 12** shows that use of a bipolar hook electrode (top panel) or a single electrode lead (bottom panel) can provide a LFBS and produce post-stimulation block.

### DESCRIPTION OF THE INVENTION

The use of numerical values in the various ranges specified in this application, unless expressly indicated otherwise, are stated as approximations as though the minimum and maximum values within the stated ranges are both preceded by the word "about". In this manner, slight variations above and below the stated ranges can be used to achieve substantially the same results as values within the ranges. Also, unless indicated otherwise, the disclosure of these ranges is intended as a continuous range including every value between the minimum and maximum values. For definitions provided herein, those definitions refer to word forms, cognates and grammatical variants of those words or phrases.

The figures accompanying this application are representative in nature, and should not be construed as implying any particular scale or directionality, unless otherwise indicated. For purposes of the description hereinafter, the terms "upper", "lower", "right", "left", "vertical", "horizontal", "top", "bottom", "lateral", "longitudinal" and derivatives thereof shall relate to the invention as it is oriented in the drawing figures. However, it is to be understood that the invention may assume various alternative variations and step sequences, except where expressly specified to the contrary. Hence, specific dimensions and other physical characteristics related to the embodiments disclosed herein are not to be considered as limiting.

As used herein, the term "comprising" and like terms are open-ended. The term "consisting essentially of" limits the scope of a claim to the specified materials or steps and those that do not materially affect the basic and novel characteristics of the claimed invention. The term "consisting of" excludes any element, step, or ingredient not specified in the claim.

As used herein, the terms "a" and "an" refer to one or more.

As used herein, the term "patient" is any mammal, including humans, and a "human patient" is any human.

As used herein, the terms "communication" and "communicate" refer to the receipt, transmission, or transfer of one or more signals, messages, commands, or other type of data. For one unit or device to be in communication with another unit or device means that the one unit or device is able to receive data from and/or transmit data to the other unit or device. A communication can use a direct or indirect connection, and can be wired and/or wireless in nature. Additionally, two units or devices can be in communication with each other even though the data transmitted can be modified, processed, routed, etc., between the first and second unit or device. For example, a first unit can be in communication with a second unit even though the first unit passively receives data and does not actively transmit data to the second unit. As another example, a first unit can be in communication with a second unit if an intermediary unit processes data from one unit and transmits processed data to the second unit. It will be appreciated that numerous other arrangements are possible. Any known electronic communication protocols and/or algorithms can be used such as, for example, TCP/IP (including HTTP and other protocols), WLAN (including 802.11a/b/g/n and other radio frequency-based protocols and methods), analog transmissions, Global System for Mobile Communications (GSM), 3G/4G/LTE, BLUETOOTH, ZigBee, EnOcean, TransferJet, Wireless USB, and the like known to those of skill in the art.

As used herein, "electrical communication," for example in the context of transmitting electrical pulses from a pulse generator to an electrode refers to sending an electrical pulse produced by a pulse generator to a skin surface electrode, an electrode lead, a magnetic coil, or like devices capable of generating electrical current to stimulate a nerve or neuron as described herein, typically through an electrically-conductive lead, such as a wire.

As used herein, the "excitation threshold" (T) of a nerve or neuron is the minimum level to which a neuron and/or nerve membrane must be depolarized to initiate an action potential, resulting in excitation of the nerve or neuron, e.g., initiation of an action potential and propagation of the action potential, and thereby propagation of a signal in the nerve or neuron. The terms "nerve" and "neuron" are used interchangeably herein, particularly with reference to excitation thresholds, though one of skill in the art will appreciate that neuron refers to the cell body at which an action potential is generated and nerve refers to the axon along which an action potential is conducted. Stimulation parameters sufficient to excite a neuron will be considered suitable to excite or propagate an action potential in a nerve. Depolarization of a nerve or neuron membrane potential results in an increase in the membrane voltage, for example from -70 millivolts (mV) to up to +40 mV. The excitation threshold may depend on the frequency at which the stimulation is delivered. For example, 0.1 mA delivered at 1 kHz may depolarize a neuron to a point at or above the neuron's excitation threshold, while the same intensity delivered at 10 Hz may not depolarize the neuron to the same degree.

As used herein, the term "super-threshold depolarization" or "super-threshold stimulation" means a stimulation sufficient to increase membrane voltage of a nerve or neuron from resting membrane potential (e.g., -70mV) to a level at or above the excitation threshold, such that the nerve or neuron becomes excited, e.g., such that an action potential is initiated or conducted. It is noted that in the same nerve trunk, the motor and sensory nerve fibers may have different excitation thresholds; however, a membrane excitation threshold can be in the range of from -55mV to -45 mV, all subranges therebetween inclusive. For the same sensory nerve, the excitation thresholds for inducing paresthesia or pain are also different. Therefore, super-threshold as used herein means that stimulation intensity is at or above the level to induce muscle contraction, paresthesia, or pain. Super-threshold stimulation as described herein can increase the membrane voltage from resting (-70 mV) to a voltage equal to or greater than -55 mV.

The "intensity" of an electrical pulse is proportional to, and refers to either the voltage or current (e.g., milliAmperes or mA) applied to the nerve or neuron, with an increased intensity being proportional to an increased voltage or an increased current applied to the nerve or neuron. Intensity may be measured as, or proportional to, electrical power, e.g., Watts.

Provided herein is a method of blocking a nerve or neuron, by applying a super-threshold electrical stimulation to the nerve or neuron. An intensity of the electrical stimulation that is applied to the nerve or neuron is greater than an excitation threshold of the nerve or neuron (e.g., depolarizing membrane voltage to equal to or greater than about - 55mV), such that an action potential is generated, and is referred to herein as "super-threshold" stimulation. This super-threshold stimulation is applied to the nerve or neuron for a length of time sufficient to produce a block of nerve conduction or neuronal excitation. The block induced by the super-threshold electrical stimulation can include a post-stimulation block.

As used herein, "post-stimulation block" refers to a nerve block that extends past the cessation of the electrical stimulation, and can, depending on the duration and intensity of the electrical stimulation, persist from seconds to hours, days, weeks, months, or years, including increments therebetween. The post-stimulation block can last at least 1 minute. The post-stimulation block can be maintained after a cessation of stimulation for at least 1 minute, optionally at least 5 minutes, 10 minutes, 15 minutes, or 30 minutes, at which time stimulation can be re-applied. The stimulation that is re-applied can be the same or different intensity and frequency as compared to the initial parameters used to initiate the block. This may be due to movement of ions and the possibility of an increase in excitation threshold achieved through super-threshold stimulation, which, without wishing to be bound by the theory, occurs by the reallocation of sodium and potassium during the application of super-threshold stimulation. After post-stimulation block is achieved, the frequency and/or intensity of the electrical stimulation can be altered to maintain or re-initiate the block. That is, after achieving post-stimulation block, the frequency of the stimulation can be increased or decreased, and/or the intensity of the stimulation can be increased or decreased.

The super-threshold stimulation can include electrical pulses (including magnetic stimulation capable of generating an electrical current) that can have any suitable characteristic, so long as the stimulation is super-threshold stimulation. As such, the terms "electrical stimulation" and "electrical pulses" are used interchangeably herein. As will be recognized by a person of skill in the art, characteristics of the electrical pulses, including, without limitation, amplitude (magnitude or size of a signal voltage or current), voltage, amperage, duration, frequency, polarity, phase, relative timing and symmetry of positive and negative pulses in biphasic stimulation, and/or wave shape (e.g., square, sine, triangle, sawtooth, or variations or combinations thereof) may be varied in order to provide a desired super-threshold stimulation and resultant post-stimulation blocking in a patient or class of patients. So long as other characteristics of the electrical signals (e.g., without limitation, amplitude, voltage, amperage, duration, polarity, phase, relative timing and symmetry of positive and negative pulses in biphasic stimulation, and/or wave shape) are within useful ranges, modulation of the pulse frequency will achieve the desired result of super-threshold induced blocking of a nerve or neuron.

Turning to **FIGS**. **1A** **and** **1B**, these figures show non-limiting examples of how to initiate super-threshold nerve block and then maintain the block for a long period of time. Nerve block can be initiated by super-threshold stimulation of a certain duration, and the block will persist after ending the stimulation (e.g., post-stimulation block). The post-stimulation block period can be extended by applying additional stimulation after the initiation of the block. The additional stimulation can be of the same or reduced intensity and applied either intermittently or continuously, or at a different frequency (**FIG. 1A**). Furthermore, the post-stimulation block can also be initiated by single or multiple stimulation pulses at an extreme super-threshold intensity and be maintained by multiple single pulses at a reduced intensity applied at regular or irregular time points (**FIG. 1B**).

One characteristic of the electrical signals used to produce a desired response, as described above, is the frequency of the electrical pulse. Although effective frequency ranges (e.g., frequencies able to produce a stated effect) may vary subject-to-subject, and the controlling factor is achieving a desired outcome, certain, non-limiting exemplary ranges may be as follows, with the proviso that the stimulation, or pulses, evoke an action potential in the target nerve/neuron. For blocking nerves, useful frequencies may range above 1 Hz (Hertz), from approximately 1 Hz to less than 4 kHz (kilohertz), or from 1 Hz to 1.5 kHz, or from 1 Hz to 1.3 kHz, or from 100 Hz to 1 kHz, optionally from 100 Hz to 500 Hz, all subranges therebetween inclusive. Effective stimulation may be delivered at 100 Hz, 500 Hz, 1 kHz, 1.5 kHz, or any value therebetween, or in a range of 1 Hz to less than 4 kHz, 1 Hz to 1.3 kHz, 1 Hz to 1.5 kHz, 100 Hz to 1 kHz, or 100 Hz to 500 Hz, all subranges therebetween inclusive. While nerve block obtained with super-threshold high frequency stimulation (≥ 4 kHz) is known, it was heretofore unknown that super-threshold stimulation in the low kilohertz range (e.g., < 4 kHz) or below kilohertz range (1-1000 Hz) could induce nerve block.

As indicated above, super-threshold electrical pulses are determined by the intensity of electrical stimulation, which in a medium of stable or relatively stable resistance, such as mammalian tissue, can be characterized as relating to current (*I*, typically measured in mA), or voltage (*V*, typically measured in mV), based on Ohm's Law. It should, therefore, be understood that the intensity of the stimulation is a matter of both *V* and *I*, and as such, both are increased, e.g., proportionally or substantially proportionally, with increased intensity of stimulation. As such, one characteristic of the pulses is the current that is applied to produce a super-threshold stimulation that is capable of nerve blocking. Super-threshold stimulation can be achieved in a typical range of from 0.1 mA to 100 mA, from 0.5 mA to 50 mA, from 0.5 mA to 5 mA, all subranges therebetween inclusive. Stimulation can be applied at 0.1 mA, 0.5 mA, 1 mA, 2 mA, 3 mA, 4 mA, 5mA, 10 mA, 50 mA, 100 mA, or any value therebetween. Super-threshold nerve stimulation can be achieved in a typical range of from 1 mV to 500 V.

Breaks, or periods where no electrical stimulation is applied, or is applied less frequently than necessary to achieve the post-stimulation block, can be introduced. When breaks or periods where no electrical stimulation are applied, the breaks or periods can be at least 10 seconds, optionally at least 1 minute, optionally at least 5 minutes, 10 minutes, 15 minutes, 30 minutes, or 180 minutes, wherein the block of nerve conduction or neuron excitation is maintained during the breaks or periods. Stimulation can then be reintroduced to maintain blockage. This reintroduced stimulation can be the same as originally applied, or of an increased or reduced intensity and/or an increased or reduced frequency compared to the stimulation providing the initial block.

Super-threshold stimulation can be measured in terms of excitation threshold (T) of a nerve or neuron, which allows for use of a metric that can be applied across patients no matter the individual variations in current and/or voltage necessary to induce excitation in a given patient. For example, and without limitation, reference can be made to excitation of 5T, or five-times the excitation threshold for a nerve of interest in a given patient, rather than to a specific combination of current/voltage. The stimulation that can be applied to a nerve in a patient to generate nerve block is 1T, 2T, 3T, 4T, 5T, 10T, 20T, 30T, 40T, 50T, 60T, 70T, 80T, 90T, 100T, or higher, all values therebetween inclusive.

As described below, the waveform of the pulses may vary, so long as the desired super-threshold blocking effect is realized. One skilled in the art will appreciate that other types of electrical stimulation may also be used in accordance with the present invention. Monophasic or biphasic stimuli, or a mixture thereof, may be used. Damage to nerves by the application of an electrical current may be minimized, as is known in the art, by application of biphasic pulses or biphasic waveforms to the nerve(s), as opposed to monophasic pulses or waveforms that can damage nerves in some instances of long-term use. "Biphasic current," "biphasic pulses," or "biphasic waveforms" refer to two or more pulses that are of opposite polarity that may be of equal or substantially equal net charge (hence, biphasic and charge balanced), and may be symmetrical, asymmetrical, or substantially symmetrical. This is accomplished, for example, by applying through an electrode one or more positive pulses, followed by one or more negative pulses, typically of the same amplitude and duration as the positive pulses, or vice versa, such that the net charge applied to the target of the electrode is zero, or approximately zero. For charge-balanced biphasic stimulation, the opposite polarity pulses may have different amplitudes, profiles, or durations, so long as the net applied charge by the biphasic pulse pair (the combination of the positive and negative pulses) is approximately zero.

The waveform may be of any useful shape, including without limitation: sine, square, rectangular, triangular, sawtooth, rectilinear, pulse, exponential, truncated exponential, or damped sinusoidal. The pulses may increase or decrease over the stimulation period. The waveform can be rectangular. The super-threshold pulses may be applied continuously or intermittently as needed. As indicated below, super-threshold stimulation of a nerve or neuron at certain voltages or currents for certain time periods elicits post-stimulation nerve blockage. Therefore, the super-threshold stimulation may be applied for very short intervals (e.g., 5 seconds to 70 seconds), short intervals (e.g., 1-10 minutes), or longer intervals (e.g., 30 minutes, 360 minutes or even longer, for example days, weeks, months, or even years) to achieve shorter-lasting blockage/relief in terms of at least 10 seconds, 30 seconds, 1 minute, 2 minutes, 3 minutes, 5 minutes, 10 minutes, or 180 minutes, or achieve longer-lasting blockage/relief, in terms of hours, days, weeks, months, or years. The stimulation can be applied for at least 5 seconds, 30 seconds, at least 70 seconds, or at least 5 minutes. The stimulation can be applied for 30 minutes to 2 hours, all subranges therebetween inclusive. The stimulation can be applied for at least 70 minutes, at least 80 minutes, or at least 90 minutes. The stimulation can be applied intermittently (that is, the pulses are turned on and off alternately during a stimulation interval for any time period) during continuous or interval stimulation protocols. For example, the stimulation may be applied for 5 seconds on and 5 seconds off over an interval of, for example, 1-10 minutes or longer (e.g., hours, days, weeks, months, years). Other examples of intermittent application of pulses may be 1-90 seconds on and 1-90 seconds off over up to a 360 minute time period or longer. So long as other pulse parameters (e.g., intensity and frequency) are within acceptable limits (e.g., those known to not to cause damage and/or long-term injury to a neuron or nerve), the inhibition is temporary and does not damage the involved neurons/nerves. For example, intermittent application of pulses may be continuous, that is, for as long as the pulses are having the desired effect, and for as long as the patient desires (e.g., is not undesirably painful, or harmful to the patient). In one aspect, the stimulation is provided continuously, for example, to treat severe symptoms, or any symptom that does not respond to intermittent, short-term stimulation to the degree desired by a clinician or the patient.

Also provided herein is a method of controlling a physiological process, such as a urological or gastrointestinal process, by applying super-threshold electrical stimulation to a patient to block nerve conduction. The physiological process can be micturition. The physiological process can also be defecation. U.S. Patent No. 8,805,510, incorporated herein by reference in its entirety, describes physiological processes that can be modulated and/or controlled by administration of electrical stimulation as described herein, including urinary incontinence, overactive bladder, urine retention and voiding dysfunction, detrusor sphincter dyssynergia, fecal incontinence, constipation, irritable bowel syndrome, sexual dysfunction in both men and women, premature ejaculation, decreased sexual sensation, an orgasm, urethral pain, prostate pain, vulvodynia, anal pain, rectal pain, and bladder pain. Such conditions can result from neurological impairment or from other diseases or conditions. For example, urinary retention and/or incontinence can result from spinal cord injury or stroke, or damage caused by trauma, disease (e.g., multiple sclerosis) and/or congenital defects. Circumstances where one or more of the conditions is caused by spinal cord trauma, or other injury that reduces and/or eliminates sensation, such as pain sensation, may benefit from the methods disclosed herein, as the patient will be less likely to experience pain from the initial excitation that accompanies the super-threshold stimulation-induced block.

The method can include the step of applying super-threshold electrical stimulation to a patient's pudendal nerve or a branch thereof. The pudendal nerve originates in the sacral plexus and derives its fibers from the first, second, third, and fourth sacral nerves (S1, S2, S3, S4). The pudendal nerve passes between the piriformis and coccygeus muscles and leaves the pelvis through the lower part of the greater sciatic foramen. The nerve then crosses the spine of the ischium, and reenters the pelvis through the lesser sciatic foramen. The pudendal nerve accompanies the internal pudendal vessels upward and forward along the lateral wall of the ischiorectal fossa, and is contained in a sheath of the obturator fascia termed the pudendal canal. The pudendal nerve gives off the inferior rectal nerves. It then divides into two terminal branches: the perineal nerve, and the dorsal nerve of the penis (males) or the dorsal nerve of the clitoris (in females). The inferior anal nerves branch off shortly after passing through the greater sciatic foramen. The dorsal nerve of the penis or dorsal nerve of the clitoris are the more superficial terminal branch of the pudendal nerve while the perineal nerve is deeper terminal branches of the pudendal nerve, traveling into the deep perineal pouch.

The pudendal nerve carries both sensory (afferent) and motor (efferent) signals. It innervates, among other things, the anal and external urethral sphincters. It also innervates the penis and clitoris, bulbospongiosus and ischiocavernosus muscles, and areas around the scrotum, perineum, and anus. At sexual climax, peristaltic action of muscles in the reproductive ducts and accessory glands (e.g., seminal vesicles, prostate and Cowper's (bulbourethral) glands), along with spasms in the bulbospongiosus and ischiocavernous muscles result in ejaculation in the male. Spasms in the bulbospongiosus and ischiocavernous muscles accompany most of the feelings of orgasm in both sexes.

The method can be used to control micturition. Micturition, also called voiding or urination, is the act of emptying the bladder. In humans, when about 200 ml of urine has accumulated, distension of the bladder wall typically activates stretch receptors, triggering a visceral reflex arc. Afferent impulses are transmitted to the sacral region of the spinal cord, and efferent impulses return to the bladder via the parasympathetic pelvic nerves, causing the detrusor muscle of the bladder to contract and the internal sphincter of the bladder to relax. As the contractions increase in intensity, they force stored urine through the internal sphincter into the upper part of the urethra. Afferent impulses are also transmitted to the brain, so one feels the urge to void at this point. Because the external urethral (urinary) sphincter is voluntarily controlled, a person can choose to keep it closed and postpone bladder emptying temporarily. On the other hand, if the time is convenient, the voluntary sphincter can be relaxed, allowing urine to be expelled from the bladder. When one chooses not to void, reflex bladder contractions subside within a minute or so and urine continues to accumulate. After 200-300 ml more has collected, the micturition reflex occurs again and, if urination is delayed again, is damped once more.

Thus, normal bladder activity is typically divided into two phases. In the first phase, the "storage phase," the bladder detrusor is quiet and the external urethral sphincter (EUS) is closed. In the second phase, the "voiding phase," the bladder detrusor contracts and the EUS is (voluntarily) relaxed, permitting urine to flow out of the urethra. In patients with neurological damage affecting the micturition process, this process is disrupted, leading to, for example, incontinence or retention.

Incontinence is the inability to control micturition. Incontinence typically is a result of emotional problems, physical pressure during pregnancy, or nervous system problems, such as stroke or spinal cord lesions.

In urinary retention, the bladder is unable to expel its contained urine. Urinary retention is common after general anesthesia has been given (it seems that it takes a little time for the smooth muscles to regain their activity). Urinary retention in men often reflects prostate hypertrophy, narrowing the urethra, making it difficult to void. Stretching of the bladder wall by urine causes sensory impulses to be transmitted to the sacral region of the spinal cord. Motor impulses are delivered to the bladder detrusor muscle and the internal sphincter via parasympathetic fibers of the pelvic nerves. The pudendal nerve serves the striated muscle fibers of the external urethral sphincter.

Defecation proceeds by a similar manner as micturition. Sensory and motor control of defecation travels through the pudendal nerve. The rectum usually is empty. When feces are forced into the rectum by mass movement, the rectal wall is stretched, initiating the defecation reflex. In the defecation reflex, the walls of the sigmoid colon and rectum contracts and the anal sphincters relax, forcing the feces into the anal canal. The brain, however, decides whether the passage of feces should be temporarily stopped. If they are stopped, the rectal walls relax, until another mass-movement initiates another defecation reflex.

Patients with supra-sacral spinal cord injuries typically have no voluntary control over the micturition, defecation and ejaculatory processes. For example, after spinal cord injury (SCI) incontinence occurs frequently due to detrusor overactivity. Meanwhile, the bladder also does not empty well due to detrusor sphincter dyssynergia (DSD) resulting in a large residual volume of urine. Thus, the management of bladder function after SCI is a challenging task, because it requires inhibition of detrusor overactivity during urine storage and induction of a large amplitude bladder contraction at the same time relaxation of the EUS to empty the bladder. Current treatment for bladder dysfunction after SCI has either limited success or requires major invasive spinal surgery to implant stimulating electrodes on spinal roots. Intermittent urethral catheterization is the most common method for managing urinary tract dysfunction. However, it can lead to frequent bladder infections. Methods and devices disclosed herein may be of particular use in individuals with SCI, as the methods can be utilized to assist in control of micturition and defecation, without the concern that the super-threshold stimulation would result in undesirable pain.

Also provided herein is a method of controlling micturition and/or defecation. The method includes applying a first electrical stimulation to the left pudendal nerve or branches thereof and a second electrical stimulation to the right pudendal nerve or branches thereof of a patient. The first and second electrical stimulation can be of an intensity that is greater than an excitation threshold of the pudendal nerve, and can include any parameters and characteristics disclosed herein as being suitable for providing nerve block by LFBS. The stimulation can be applied for a length of time sufficient to produce a conduction block of both pudendal nerves either during or after ending the first and/or second electrical stimulation, thereby inhibiting contraction of the external urethral sphincter (EUS) and/or external anal sphincter (EAS).

Following the block, or during the blocking stimulation, when the EUS/EAS is relaxed, a third electrical stimulation to the left or right pudendal nerve can be applied at a location central to the blocked pudendal nerve site to induce bladder or colon/rectal contraction. Alternatively, again either during or immediately after ending the first and/or second electrical stimulation when the EUS/EAS is relaxed, a third electrical stimulation can be applied to a sacral spinal root (S1, S2, S3, or S4) to induce bladder or colon/rectal contraction. Alternatively, again either during or immediately after ending the first and/or second electrical stimulation when the EUS/EAS is relaxed, a third electrical stimulation can be applied to the spinal cord by epidural electrodes, or skin surface electrodes or electromagnetic coils to induce bladder or colon/rectal contraction. Alternatively, again either during or immediately after ending the first and/or second electrical stimulation when the EUS/EAS is relaxed, applying a third electrical stimulation to pelvic nerve to induce bladder or colon/rectal contraction. Alternatively, and again either during or immediately after ending the first and/or second electrical stimulation and during the EUS/EAS relaxation, pressure can be applied to the abdominal area to produce bladder or colon/rectal pressure. With regard to the steps involving stimulation, devices as described herein (discussed below) can include memory having programming instructions stored thereon, the programming instructions causing a processor to perform (through control of, e.g., a pulse generator) the various stimulations described above.

Also disclosed herein is a method of controlling micturition and/or defecation in a patient by applying first electrical stimulation to either left or right pudendal nerve or branches thereof of a subject, wherein the electrical stimulation is of an intensity that is greater than an excitation threshold of the pudendal nerve for a length of time sufficient to produce a block of pudendal nerve conduction either during or after ending the electrical stimulation, thereby inhibiting contraction of the external urethral sphincter (EUS) and/or external anal sphincter (EAS). The method further includes, either during or immediately after ending the first electrical stimulation when the EUS/EAS is relaxed, applying a second electrical stimulation to the same side of the blocked pudendal nerve at a location central to the blocked pudendal nerve site to induce bladder or colon/rectal contraction. Alternatively, and either during or immediately after ending the first electrical stimulation when the EUS/EAS is relaxed, a second electrical stimulation can be applied to a sacral spinal root (S1, S2, S3, or S4) to induce bladder or colon/rectal contraction. Alternatively, and again either during or immediately after ending the first electrical stimulation when the EUS/EAS is relaxed, a second electrical stimulation can be applied to the spinal cord by epidural electrodes, or skin surface electrodes or electromagnetic coils to induce bladder or colon/rectal contraction. Alternatively, and again either during or immediately after ending the first electrical stimulation when the EUS/EAS is relaxed, a second electrical stimulation can be applied to the pelvic nerve to induce bladder or colon/rectal contraction. Alternatively, and again either during or immediately after ending the electrical stimulation when the EUS/EAS is relaxed, pressure can be applied to the abdominal area to produce bladder or colon/rectal pressure. With regard to the steps involving stimulation, devices as described herein (discussed below) can include memory having programming instructions stored thereon, the programming instructions causing a processor to perform (through control of, e.g., a pulse generator) the various stimulations described above.

While urological and gastrointestinal processes are exemplified above, those of skill in the art will appreciate that the methods and devices described herein can be useful for treating other systems/processes where blockage of nerve conduction may prevent, improve, resolve, or reverse a condition or disease. For example, and without limitation, blockage of conduction of afferent pain fibers, blockage of the splanchnic nerve for treating heart failure (*see* Fidum et al., Splanchnic nerve block for acute heart failure, Circulation, 2018, Vol. 138, pp. 951-53) and blockage of conduction of the vagus nerve for treating obesity (*see* Sarr et al., The EMPOWER Study: Randomized, prospective, double-blind, multicenter trial of vagal blockage to induce weight loss in morbid obesity, Obes Surg, 2012) can be achieved with the devices and methods disclosed herein.

Also provided herein are devices and systems for applying super-threshold stimulation in a manner sufficient to induce post-stimulation nerve/neuron block. **FIG. 2A** provides a general schematic of one non-limiting embodiment or aspect of an electrical stimulation device **10** useful in the methods described herein. The device **10** includes a power supply or pulse generator **20**. The power supply/pulse generator **20** may be fixed output, or may be adjustable, for example within a useful range as described herein. The device **10** includes a first conductive lead **30** and, optionally, a second conductive lead **35**. While **FIG. 2A** shows leads **30, 35** in contact with nerve **37**, those of skill in the art will appreciate that conductive leads need not be in such close proximity such that they contact the nerve or neuron of interest. For example, and without limitation, leads (**30 and/or 35**) can be placed within 10 mm, 9 mm, 8 mm, 7 mm, 6 mm, 5mm, 4mm, 3mm, 2mm, or 1mm of a nerve or neuron of interest, all subranges therebetween inclusive.

A benefit of the present methods, and the use of super-threshold stimulation, is the option to utilize one or more leads that need not be cuffed (e.g., need not be placed on a nerve or neuron, but instead may be placed merely in proximity to the nerve or neuron of interest). Conductive leads **30, 35** can be directly wired to power supply/pulse generator **20**, or may each comprise multiple leads and electrical connectors, fasteners, terminals, or clips to produce a contiguous electrical connection between the power supply/pulse generator **20** and the end of the leads. One or more leads for grounding the circuit (not shown) can also be provided, and can be attached to the patient's body. Skin **38** is also shown, and as such the device **10** is external and can be a hand-held or body-worn device - held in place by a belt or strap, such as by a hook and loop fastener band, though optionally, the device **10** can be an implantable device (described in more detail below). In **FIG. 2A**, the leads are of opposite polarity and, together, form a circuit for application of any electrical waveform described herein. Alternative designs, with different leads, probes, electrodes, or electrical contacts, or combinations thereof will be apparent to those of ordinary skill. As used herein, an "electrical contact" is inclusive of any structure useful for directly applying an electrical current to a nerve or tissue in a patient, such as to the skin of a patient. Structures for producing a magnetic field, and therefore an electrical current via induction, are not considered to be electrical contacts. Nevertheless, induction probes, that is structures capable of generating a magnetic field capable of producing an electrical current, can be used to produce the electrical pulses described herein.

**FIG. 2B** depicts schematically another aspect of a device **10** for nerve block, which, like the device of **FIG. 2A**, has an external power supply. In **FIG. 2B**, like reference numbers as compared to reference numbers of **FIG. 2A**, refer to like elements of the device **10**. However, surface electrodes **31** and **36** are utilized, and stimulation is transcutaneous. In an alternative aspect, not shown, surface electrodes **31** and **36** are replaced by electromagnets for magnetic induction stimulation of impulses in nerve **37**.

**FIG. 2C** depicts a further aspect of the nerve block device **110** that is implanted, and includes an implantable housing **112**. The housing **112** contains various subunits of the device, including a power supply/pulse generator **120** connected to a first lead **130** and a second lead **135** for stimulating a nerve **137**. Skin **138** is depicted for context. The housing may be composed of any biocompatible material as are known in the medical fields for use in such implantable devices, such as a plastic, metal, carbon fiber, or ceramic material, or a polymer-coated material, such as a metal or plastic housing coated with a biocompatible polymer or hydrogel. The housing **112** also contains various connected subunits of the device **110**, including a processor **140**, a storage module **142** including transient data storage (e.g., RAM), and non-transient data storage, such as flash memory or a solid-state drive, and a battery **144** that is optionally rechargeable by electromagnetic induction. The processor **140** can also be connected to a wireless communications module **150** for communicating wirelessly, e.g., by near-field communication, or by BLUETOOTH, Wi-Fi, or over a cellular network, with an external computer or computer network, such as a smartphone, tablet, laptop, personal computer, smart watch, workstation, server, or computer network.

The devices of **FIGS. 2A-2C** can be battery-powered, and optionally the battery is rechargeable. Where the device is implanted, the device can be recharged by wireless, e.g., magnetic induction recharging methods, as are known. The devices of **FIG. 2A** **and/or** **FIG. 2B** also can include a communications interface, such as a wireless communications interface or module, for transmitting data, and for receiving instructions from a separate computing device, such as from a controller app or software on a smartphone, tablet, laptop, personal computer, workstation, server, or computer network. As would be appreciated by those of ordinary skill in the fields of computer and software engineering, a multitude of potential device and system configurations and implementation schemes can be used to control devices and systems that provide electrical stimulation and nerve block as described herein.

Referring to **FIG. 2C**, but equally applicable to any aspect of the device, e.g., device **10** of **FIG. 2A** **and/or** **FIG. 2B**, the device **110** comprises a controller for executing functions related to electrical pulse output of the power supply. In some examples, a controller is a central processing engine including a baseline processor, memory, and communications capabilities. For example, the controller can be any suitable processor comprising computer readable memory and configured to execute instructions either stored on the memory or received from other sources. Computer readable memory can be, for example, a disk drive, a solid-state drive, an optical drive, a tape drive, flash memory (e.g., a non-volatile computer storage chip), cartridge drive, and control elements for loading new software.

In some examples, the controller includes a program, code, a set of instructions, or some combination thereof, executable by the processor for independently or collectively instructing the device to interact and operate as programmed, referred to herein as "programming instructions". In some examples, the controller is configured to issue instructions to the power supply/pulse generator to initiate super-threshold electrical pulses, and to control output parameters of the power supply in a manner sufficient to induce nerve/neuron block, optionally post-stimulation block, as described throughout this disclosure (e.g., super-threshold stimulation, altering stimulation parameters once block has been achieved, and the like). Those of skill in the art will appreciate that a processor associated with a device **10, 110** disclosed herein can be programmed to deliver suitable super-threshold stimulation as described generally throughout this disclosure. In any case, the controller is configured to receive and process electrical pulse parameters, either programmed into the device or from an external source, and optionally to output data obtained from the power supply as feedback to determine if the power supply is producing a desired output. Processing can include applying filters and other techniques for removing signal artifacts, noise, baseline waveforms, or other items from captured signals to improve readability.

Further to the above, the device **10, 110** can include programming instructions that, when executed by the processor **140**, cause the power supply/pulse generator **120** to apply electrical stimulation at an intensity at or above an excitation threshold of the nerve/neuron (e.g., at or above -55 mV) for a time sufficient to cause a block and/or a post-stimulation block in the nerve or neuron, or to deliver a physiological outcome as described above. Useful parameters are described above, but can include stimulation at, for example and without limitation, 100 Hz, 500 Hz, 1 kHz, from 1 Hz to less than 4 kHz or from 1 Hz to 1.5 kHz, from 1 Hz to 1.3 kHz, from 100 Hz to 1.5 kHz, from 100 Hz to 500 Hz, from 500 Hz to 1.5 kHz, all values subranges therebetween inclusive, at an intensity of, for example and without limitation, 1 mA, 3 mA, 9mA, 12 mA, 15mA, or from 0.1 mA to 50 mA, for a duration of seconds to days, all subranges therebetween inclusive for all parameters.

As also described previously, the processor **140** can thereafter instruct the power source/pulse generator **120** to apply a first decreased or increased intensity and/or frequency electrical stimulation following initiation of block. The controller can be programmed or configured to, once block of nerve conduction or neuron excitation is achieved, instruct the pulse generator to change the intensity and/or frequency of the electrical stimulation. Various sensors and devices can be utilized to determine whether block has been achieved. For example, as described above and illustrated in the examples below, a device can include more than one contact or lead. One of the contacts/leads can be located proximally of the blocking contact/lead, and blocking can be determined by whether a stimulation pulse applied proximally of the block results in transmission of an action potential distally of the location of the blocking contact/lead.

A device **10, 110** can include multiple channels and multiple electrode leads/contacts, to provide a blocking stimulation and an excitatory stimulation, such that the device **10, 110** can, through application of stimuli at various times, locations, and intensities, induce micturition, defecation, and/or retention. Stimulation parameters and electrode lead/contact placement for inducing such physiological actions are disclosed in U.S. Patent No. 9,623,243, the contents of which are incorporated herein by reference in their entirety. Briefly however, for example and without limitation a device **10, 110** as described herein, whether implanted, wearable, or otherwise disposed externally, can include multiple channels, one (or more) channels for providing a blocking stimulation as described herein, and one (or more) channels for providing a stimulation that causes, for example and without limitation, contraction of the EUS or the anal sphincter (e.g., stimulation at 0.5 Hz to 15 Hz) and/or contraction of the bladder or colon/rectum (e.g., 15 Hz to 50 Hz), such that the device can control micturition and/or defecation. Device **10, 110** can include multiple channels for providing a blocking stimulation as described herein, for example, and without limitation, to bilateral pudendal nerves (e.g., two branches of the pudendal nerve on contralateral sides of the body). A device **10**, **110** useful for such treatment, e.g., a device having three output channels as described in U.S. Patent No. 9,623,243, can be used in a method including applying an electrical signal to a pudendal nerve or a branch thereof of a patient at a first point on the pudendal nerve or branch thereof, the electrical signal having an amplitude and frequency able to create a reflex that results in one or both of bladder contractions and rectal contractions; and applying a super-threshold electrical blocking signal (as described herein) distal to the first point on the pudendal nerve of the patient or a branch thereof and/or on a contralateral pudendal nerve or branch thereof, the blocking electrical signal having an amplitude and frequency able to block pudendal nerve conduction for inhibiting contraction of one or both of the external urethral sphincter and anal sphincter of the patient. The stopping and starting of the stimulation can be repeated multiple times to induce bladder or colon/rectal contraction when the pudendal nerves are blocked and the EUS and/or anal sphincter is relaxed, until the bladder or colon is fully emptied.

### Example 1

### Materials and Methods

Nine cats (5 males and 4 females; 3.3-4.2 kg; Marshall BioResources, North Rose, NY, USA) were anesthetized by isoflurane (2-5% oxygen) during surgery and switched to α-chloralose anesthesia (initial 65mg/kg i.v. with supplemental as needed) during data collection. The right cephalic vein was catheterized for administration of fluid or anesthetics. The airway was kept patent by a tracheotomy. A catheter was inserted into the right carotid artery to monitor the blood pressure. A pulse oximeter (9847V; NONIN Medical, Plymouth, MN) was attached to the tongue to monitor the heart rate and blood oxygen.

Via an abdominal incision a catheter was inserted into the distal urethra to slowly (1 ml/min) perfuse the urethra with saline and record urethral pressure increase caused by contractions of external urethral sphincter (EUS) that was induced by pudendal nerve stimulation (**FIG. 3**). The ureters were tied, cut, and drained externally. The left pudendal nerve was exposed via a 3-4 cm incision in the sciatic notch lateral to the tail for implantation of a tripolar cuff electrode (NEC113, MicroProbes Inc, Gaithersburg, MD, USA) to deliver HFBS (Stim.B in **FIG. 3**). Two hook electrodes were placed central (Stim.C) and distal (Stim.D) to the tripolar cuff electrode. The right pudendal nerve was also exposed and implanted with the same 3 sets of electrodes. Pudendal nerves were transected centrally to prevent reflex activation of the EUS (**FIG. 3**). Stimulus pulses (20 Hz, 0.2 ms) generated by a stimulator (Grass S88, Grass Technologies, RI, USA) was delivered via a stimulation isolator (SIU5, Grass Technologies, RI, USA) to the hook electrodes (Stim.C or Stim.D) to induce >30 cmH₂O urethral pressure. LFBS (1kHz, 500 Hz, or 100 Hz) square waveform without a pulse interval, *see* **FIG. 3**) generated by a computer running a LabView program (National Instrument, TX, USA) was delivered via a stimulation isolator (A395, World Precision Instruments, FL, USA) to the tripolar cuff electrode to block pudendal nerve conduction and suppress EUS contractions induced by Stim.C (**FIG. 3**). As initial nerve firing is not a concern when, for example, a patient has had a spinal cord injury, the 1 kHz LFBS can be applied at a super-threshold intensity (e.g., 5T, 10T, 20T, 30T, 40T, or 50T as shown in **FIGS. 4A-4B**) that will significantly shorten the time to achieve a nerve conduction block.

### Results

**FIGS. 4A** **and** **4B** show that stimulation delivered at 1 kHz at 5T (0.5 mA) (here, T = excitation threshold of the nerve), 10T (1 mA), 20T (2 mA), 30T (3mA), 40T (mA), and 50T (5 mA) can produce nerve block and a significant post-stimulation block. **FIG. 5** shows that blocking can be accomplished at a lower frequency (500 Hz), when the intensity is 90T (9 mA) when the stimulation is delivered for 70 seconds, and **FIG. 6** shows that blocking can be accomplished at an even lower frequency (100 Hz) at an intensity of 90T (9 mA) when the stimulation is delivered for 30 seconds.

### Discussion

The results presented here show that LFBS, delivered at an intensity above the excitation threshold of a nerve or neuron (e.g., 5T, 10T, 20T, 30T, 40T, 50T), can quickly induce blocking and post-stimulation block in the nerve or neuron.

### Example 2

### Materials and Methods

### Experimental Protocol

A total of 10 cats (5 females and 5 males, 2.9-3.7 kg) were used in this study. The animals were anesthetized initially with isoflurane (2-5% in oxygen) during surgery and then switched to alpha-chloralose anesthesia (initial dose 65 mg/kg i.v. followed by supplemental doses as needed) during data collection. The right cephalic vein was catheterized for intravenous administration of fluid and drugs. A midline anterior cervical incision was used to access the airway, which was kept patent via tracheostomy. The right carotid artery was catheterized for monitoring arterial blood pressure. Oxygen saturation and heart rate were measured via a pulse oximeter (9847V, NONIN Medical, Plymouth, MN) attached to the tongue. Through an abdominal incision, the ureters were isolated, cut, and drained externally. A catheter was inserted into the urethra via a small incision in the proximal urethra. The catheter was connected to a syringe pump (SP200i; World Precision Instruments, Sarasota, FL) and a pressure transducer (BLPR2, World Precision Instruments) via a three-way stopcock to slowly (1 ml/min) perfuse the urethra and measure the urethral pressure increase caused by neutrally evoked contractions of the external urethral sphincter (EUS) (**FIG. 3**). Each pudendal nerve was exposed via a 3-4 cm incision in the sciatic notch lateral to the tail for implantation of a tripolar cuff electrode (NEC113, MicroProbes Inc, Gaithersburg, MD, USA) to deliver a biphasic stimulation waveform (Stim.B, **FIG. 3**). Bipolar hook electrodes were placed distal (Stim.D) and central (Stim.C) to the tripolar cuff electrode (**FIG. 3**). Each pudendal nerve was transected centrally to prevent reflex activation of the EUS. The nerve and electrodes were covered with warm (37 °C) mineral oil. Stimulus pulses (30 Hz, 0.2 ms) generated by a stimulator (Grass S88, Grass Technologies, RI, USA) were delivered via a stimulus isolator (SIU5, Grass Technologies, RI, USA) to the hook electrodes (Stim.C or Stim.D) to induce a EUS contraction and >30 cmH2O increase in urethral pressure. The biphasic stimulation waveform generated by a computer running a LabView program (National Instruments, TX, USA) was delivered via a stimulus isolator (A395, World Precision Instruments, FL, USA) to the tripolar cuff electrode to block pudendal nerve conduction and suppress EUS contractions (Fig.1).

### Stimulation Protocol

The intensity threshold to block pudendal nerve conduction was determined at the beginning of every experiment by applying 10 kHz HFBS for 50-60 seconds at an increasing intensity starting from 1 mA with 1 mA increments. The minimal intensity for 10 kHz HFBS to completely suppress the urethral pressure induced by stimulation at Stim.C was determined as the block threshold (T) (see **FIG. 7**, here T = the block threshold for 10 kHz stimulation to block nerve conduction). This frequency and duration of stimulation was shown in previous experiments to consistently block pudendal nerve conduction during the stimulation without causing a post-stimulation block. 10 kHz block threshold was chosen as the reference intensity in this study because 10 kHz nerve block is well known and recent studies show that 10 kHz requires a very long stimulation duration (10-30 minutes) to induce a post-stimulation block. In preliminary studies using the first 3 cats, LFBS (1 kHz, 500 Hz, or 100 Hz) was applied to the pudendal nerve at different intensities (1T, 2T or 3T) with different durations (5 secs to 3 minutes) randomly to determine if post-LFBS block could occur. In every experiment Stim.B was identified as the site of post-LFBS nerve block by showing that stimulation at the Stim.D site (30 Hz frequency, 0.2 ms pulse width, 5 secs duration) still induced >30 cmH2O EUS contractions when stimulation at the Stim.C site (30 Hz frequency, 0.2 ms pulse width, 5 secs on and 55 secs off) failed to induce EUS contractions (see **FIG. 8**). In the remaining 7 cats, the minimal intensity and duration of LFBS (1 kHz, 500 Hz, or 100 Hz) to induce a complete post-LFBS block was examined in a systemic manner. A complete post-LFBS block was defined as the suppression of at least one of the EUS contractions induced by stimulation at Stim.C (30 Hz frequency, 0.2 ms pulse width, 5 secs on and 55 secs off) to <5 cmH2O urethral pressure. The testing was started using 1 kHz LFBS of 60 secs duration applied at an increasing intensity of 1T, 2T and 3T. If a complete post-LFBS block did not occur, then the LFBS intensity was kept at 3T and the stimulation duration was increased sequentially by 30 secs in repeated tests until a complete post-LFBS was observed (see **FIG. 9**). This was then followed by 500 Hz LFBS at 1T starting at 5 secs duration. The duration was then increased sequentially in 5-10 sec increments to a maximum of 60 secs. If complete block was not elicited, the intensity was then increased to 2T at 60 sec and then to 3T if needed starting at 60 secs followed by sequential 5 second increases in duration until the complete post-LFBS block was observed. In the final series of tests, 100 Hz LFBS was initially applied at 1T intensity and 5 secs duration followed by sequential 5-10 sec increases to a maximum of 60 secs. If a complete post-LFBS block was not observed, the intensity was increased to 3T and 5 secs duration and then duration was increased sequentially in 5-10 secs steps until a complete post-LFBS block was observed. At the end of each LFBS test, a rest period (3-30 minutes) was inserted to allow the EUS contractions to fully recover before applying the next period of LFBS. 1 kHz was tested in 14 nerves (left and right in 7 cats), 500 Hz was tested in 9 nerves, and 100 Hz was tested in 6 nerves.

### Data Analysis

The recovery period was defined as the time period required for the EUS contraction pressure to reach >90% of the pre-stimulation pressure (**FIG. 10****, top panel**). The minimal LFBS stimulation durations to induce a complete post-LFBS block at different frequencies (1 kHz, 500 Hz, and 100 Hz) were compared. The data obtained under the same conditions in different animals were averaged and presented as mean + standard error. Unpaired student t-test was performed to detect significant differences (p<0.05).

### Results

At the beginning of each experiment the urethral pressure responses indicating EUS contractions induced by electrical stimulation (30 Hz intermittent, 5 secs on and 55 secs off) of the pudendal nerve at sites central (Stim C) and distal (Stim D) to the blocking electrode (Stim B) (**FIG. 3**) were elicited over a range of intensities. Stimulus intensities (0.6-1.2 V, 0.2 ms pulse width) that produced approximately equal increases in urethral pressure >30 cmH2O at both sites were used for the remainder of the experiments to elicit control responses that were tested for sensitivity to LFBS block. Preliminary studies in the first 3 cats examined the effect of LFBS at different frequencies (1 kHz, 500 Hz, or 100 Hz) and stimulus intensities. Stimulus intensities were normalized in each animal by referencing to the stimulus intensity threshold (T) at 10 kHz that produced a transient increase in urethral pressure followed by a complete block of the urethral pressure response to stimulation at Stim.C (see **FIG. 7**). As shown in **FIG. 8**, the LFBS (Stim.B: 1 kHz, 3T = 9 mA, 120 secs) induced a tonic EUS contraction during the stimulation and a complete post-LFBS block that fully suppressed EUS contractions induced by electrical stimulation (30 Hz, 0.2 ms, 0.6 V, 5 secs on and 55 secs off) via the electrode at Stim.C. The EUS contractions gradually recovered in about 5.5 minutes following the LFBS (**FIG. 8**). During the post-LFBS block, stimulation at the distal electrode (Stim.D: 30 Hz, 0.2 ms. 0.6 V, 5 secs on) induced a EUS contraction of the same amplitude as that prior LFBS (**FIG. 8**), indicating that the LFBS blocked the pudendal nerve locally at the Stim.B site but not distally at sites in the nerve, the neuromuscular junction, or due to fatigue of the EUS muscle.

The search protocol to determine the minimal stimulation intensity and duration required to induce a complete post-LFBS block after 1 kHz stimulation is shown in **FIG. 9**. As the 1 kHz LFBS of 60 secs duration increased in intensity from 1T to 3T, the post-LFBS block was first observed at 3T intensity as a 50% reduction in the EUS contraction pressure (see the top trace in **FIG. 9**). At 3T intensity, further increasing the duration of stimulation from 60 secs to 150 secs produced a complete post-LFBS block (see the bottom trace in **FIG. 9**). Therefore, the minimal stimulation intensity and duration were determined as 3T and 150 secs for 1 kHz LFBS to induce a complete nerve block (**FIG. 9**). Using the same nerve in this cat, the minimal stimulation intensity and duration for LFBS of 500 Hz (**FIG. 10****, top panel**) or 100 Hz (**FIG. 10****, bottom panel**) to induce a complete post-LFBS block were also determined. The recovery from complete block had a similar time course for both frequencies of stimulation (**FIG. 10**). Although 3T intensity was required in this cat for 1 kHz, 500 Hz, and 100 Hz LFBS to induce a complete post-LFBS block, the block was also observed at 1T or 2T intensity in the other 6 cats at different frequencies. On average (N=7 cats) the minimal stimulation duration to induce a complete block significantly (p<0.05) increased from 23±8 secs to 95±14 secs when the frequency increased from 100 Hz to 1 kHz (**FIG. 11****, top panel**) although the block occurred at various stimulation intensities (1-3T). At the minimal stimulation intensity and duration, the complete post-LFBS block induced by stimulation of different frequencies had similar complete block duration (**FIG. 11****, middle panel**) and recovery period (**FIG. 11****, lower panel**). The post-LFBS block induced at the minimal stimulation intensity and duration was fully reversible (**FIGS. 8-10**) in every experiment within the same time period (10-15 minutes on average) for the three frequencies (**FIG. 11****, middle and bottom panels**).

### Discussion

This study in cats discovered that post-stimulation block of pudendal nerve conduction could be induced by LFBS at a frequency less than 1 kHz (**FIGS. 8-10**) and at a lower frequency, a shorter stimulation duration is required to induce a complete post-LFBS block (**FIG. 11****, top panel**). The block induced by LFBS at the minimal intensity and duration is fully reversible within a similar recovery period for different frequencies (**FIG. 11****, middle and bottom panels**). These results provide valuable information for understanding the possible mechanisms underlying the block of nerve conduction by biphasic stimulation waveforms. This study also opens the opportunity for clinical applications to induce post-stimulation block at a low frequency (≤1 kHz) within 30-60 seconds (**FIG. 11****, top panel**) while the traditional 10 kHz HFBS requires a much longer stimulation duration (10-30 minutes) to achieve a post-stimulation block.

The post-LFBS block is fully reversible, which indicates that the nerve is likely not damaged by the LFBS. Therefore, it is reasonable to assume that the post-LFBS block is caused by alteration of the ionic mechanisms underlying axonal conduction. Previous computer simulation studies revealed that each stimulus pulse of the biphasic stimulation waveform can generate an inward sodium current and an outward potassium current. Therefore, it is reasonable to expect that the intracellular and extracellular ion concentrations must be changed dramatically as the LFBS continues. When LFBS is terminated, these large changes in ion concentrations must disrupt the normal transmembrane ionic gradients necessary for the generation of the action potential and cause nerve conduction block. As the transmembrane ion pumps gradually restore the normal intracellular and extracellular ion concentrations, the post-LFBS block will slowly disappear and the nerve conduction will be restored. This mechanism revealed by computer simulation studies can explain very well the post-LFBS block observed in current study. It is worth noting that the LFBS induced a tonic EUS contraction during the stimulation but blocked the nerve conduction after the stimulation (**FIGS. 8-10**). This is in dramatic contrast to the HFBS (10 kHz) that instead of inducing a tonic EUS contraction produced a nerve block during the stimulation but did not block the nerve after the stimulation (**FIG. 7**). These results indicate that the mechanisms are very different for post-LFBS block and the block induced by HFBS during the stimulation. In fact, our previous computer simulation studies did reveal a different blocking mechanism for HFBS that maintains potassium channels in a constantly open state at stimulation frequencies ≥5 kHz. This causes nerve conduction block during stimulation (**FIG. 7**) instead of the block that occurs after stimulation that is mediated by changes in transmembrane ionic gradients.

It is also important to note that at a lower frequency each stimulus pulse of the biphasic stimulation waveform (see **FIG. 3**) has a longer duration that can drive more sodium and potassium ions across the axonal membranes and therefore be more effective in changing the ion concentrations than higher frequencies of stimulation that have a shorter duration stimulus pulse. This may explain why the lower frequency (100 Hz) stimulation requires a shorter stimulation duration than the higher frequencies (500 Hz or 1 kHz) to induce a complete post-LFBS block (**FIG. 11****, top panel**). This finding also raises the question of whether a lower frequency (<100 Hz) would be effective at an even shorter duration? However, the proposed mechanism must have a minimal effective frequency because during a very long stimulus pulse, the sodium and potassium channels will open at the beginning of the pulse but will be closed before the end of the pulse due to the ion channel kinetics. Therefore, for a very low frequency (i.e., a very long stimulus pulse) the LFBS will become less effective as the inward sodium and outward potassium currents during the last phase of the long stimulus pulse will decline and have less impact on the intracellular and extracellular ion concentrations.

Although the minimal stimulation duration for producing post-LFBS block is frequency dependent, the nerve blocks induced by different LFBS frequencies have a similar time course for recovery (**FIG. 11****, middle and bottom panels**). This is expected because complete conduction block at different frequencies is probably occurring after the same change in ion concentrations induced by LFBS at the minimal intensity and duration; whereas recovery occurs after a time required for the membrane pumps to restore normal ion concentrations, which should be frequency independent because the starting point for recovery should be the same after 100 Hz to 1 kHz stimulation However, based on the ion concentration hypothesis the duration of post-LFBS block should be increased by LFBS of a high intensity or a long duration. The maximal duration of post-LFBS block induced by a strong LFBS of a long duration without causing nerve tissue damage still needs to be determined. In addition, the intensity-duration relationship for LFBS at a fixed frequency to induce a post-LFBS block also needs to be studied as well as the intensity frequency relationship for LFBS of a fixed duration. These relationships will provide more information to support or refute the ion concentration hypothesis of nerve block.

The LFBS always produces tonic nerve firing during stimulation before it can induce a post-LFBS block (**FIGS. 8-10**). Therefore, it is less likely that LFBS will be used clinically to block peripheral nerves for treatment of chronic pain. However, the post-LFBS block will be very useful in clinical applications where initial tonic nerve firing is acceptable. For example, post-LFBS block can be used to prevent detrusor sphincter dyssynergia and allow efficient voiding after spinal cord injury by blocking the pudendal nerve conduction as shown in this study. During the 2-5 minutes of complete post-LFBF pudendal nerve block (**FIG. 11****, middle panel**), the EUS relaxes to allow efficient voiding to occur at a low bladder pressure.

In summary, this study discovered a novel method to block nerve conduction using a biphasic stimulation waveform of a low (≤1 kHz) frequency. The results confirmed that post-stimulation block can be induced not only by HFBS (≥5kHz) but also by LFBS (≤1 kHz), supporting the theory that ion concentration changes may play an important role in nerve conduction block by biphasic stimulation waveform. The post-LFBS block could be used in many clinical applications where initial tonic nerve firing is acceptable, providing opportunities to develop new neuromodulation devices.

### Example 3

### MATERIALS AND METHODS

The experiment setup as shown in **FIG. 3** was also used to obtain the results shown in **FIG 12**. However, the cuff electrode (Stim.B in **FIG. 3**) was replaced by a hook electrode or by a single lead electrode to deliver LFBS to block the pudendal nerve conduction.

### RESULTS

As shown in **FIG. 12****, top panel** a bipolar hook electrode, delivering a blocking stimulation (Stim. B) at 1 kHz, 0.5 mA, 0.2 ms pulsewidth for 2.5 minutes can provide post-stimulation blocking. As also shown (**FIG. 12****, bottom panel)**, use of a single lead electrode to deliver a LFBS (100 Hz, 0.5 mA, 0.2 ms pulsewidth, for 30 seconds) can similarly provide post-stimulation blocking.

### DISCUSSION

The results presented here show that a bipolar/tripolar cuff electrode, which is highly invasive in terms of needing access in order to surround, or at least partially surround, a nerve is not required to deliver LFBS. Rather, a simple electrode lead, placed in proximity to a nerve of interest (e.g., the pudendal nerve), can provide blocking, with post-stimulation blocking, to deliver the therapeutic effects discussed herein.

Although the methods and devices have been described in detail for the purpose of illustration based on what is currently considered to be the most practical and preferred embodiments, it is to be understood that such detail is solely for that purpose and that the systems and methods are not limited to the disclosed embodiments, but on the contrary, is intended to cover modifications and equivalent arrangements that are within the spirit and scope of the appended claims. For example, it is to be understood that the present systems and methods contemplate that, to the extent possible, one or more features of any embodiment can be combined with one or more features of any other embodiment.

The following items are embodiments of the invention:
1. A method of blocking a nerve or neuron, comprising:
   applying an electrical stimulation to the nerve or neuron, wherein the electrical stimulation is of an intensity that is equal to or greater than an excitation threshold of the nerve or neuron for a length of time sufficient to produce a block of nerve conduction or neuron excitation.
2. The method of item 1, wherein the electrical stimulation is delivered at an intensity that is at least five times the excitation threshold of the nerve or neuron, optionally wherein the intensity is of 0.01 mA to 100 mA and/or 1 mV to 500 V, optionally 0.5 mA to 10 mA.
3. The method of item 1, wherein the electrical stimulation is delivered at a frequency of 1 Hz to less than 4 kHz, or 1 Hz to 1.5 kHz, or 1 Hz to 1.3 kHz, optionally from 100 Hz to 1 kHz, optionally from 100 Hz to 500 Hz.
4. The method of item 1, wherein the electrical stimulation is delivered for a period of from 5 seconds to 14 days, 1 minute to 14 days, optionally from 2 minutes to 2 hours, optionally from 1 minute to 7 days, optionally from 1 minute to 5 days, optionally from 2 minutes to 5 minutes, optionally from 5 seconds to 90 seconds.
5. The method of item 1, wherein the electrical stimulation results in the block of nerve conduction or neuron excitation for at least 10 seconds following cessation of the electrical stimulation.
6. The method of item 1, wherein the electrical stimulation comprises biphasic electrical pulses.
7. The method of item 6, wherein the biphasic pulses are symmetric between the positive and negative phases of the biphasic pulse.
8. The method of item 6, wherein the biphasic pulses are asymmetric between the positive and negative phases of the biphasic pulse.
9. The method of item 1, wherein the electrical stimulation comprises biphasic electrical pulses that are charge-balanced.
10. The method of item 1, further comprising, once block of nerve conduction or neuron excitation is achieved, stopping application of the electrical stimulation for a period of at least 10 seconds, optionally at least 1 minute, optionally at least 5 minutes, 10 minutes, 15 minutes, 30 minutes, or 180 minutes, wherein the block of nerve conduction or neuron excitation is maintained during the period and, after the period has concluded, resuming electrical stimulation of the nerve or neuron at the same or different intensity and/or at the same or different frequency to continue or prolong the block of nerve conduction or neuron excitation.
11. The method of item 1, further comprising, once block of nerve conduction or neuron excitation is achieved, maintaining the block by changing the intensity and/or frequency of the electrical stimulation, optionally by reducing the intensity of the electrical stimulation or increasing the frequency of the electrical stimulation.
12. The method of item 1, wherein the stimulation comprises biphasic pulses at an intensity of 6-9 mA and a frequency of between 100 Hz and 1 kHz, applied for from 10-60 seconds.
13. A device comprising:
   a controller;
   a pulse generator in communication with the controller; and
   an electrode lead or cuff configured to be placed near or in contact with a nerve or neuron, the electrode lead/cuff being in electrical communication with the pulse generator, wherein
   the device is configured to apply an electrical stimulation to the nerve or neuron, wherein the electrical stimulation is of an intensity that is equal to or greater than an excitation threshold of the nerve or neuron for a length of time sufficient to produce a block of nerve conduction or neuron excitation.
14. The device of item 13, wherein the pulse generator is configured to deliver electrical stimulation through the electrode lead/cuff at an intensity that is at least five times the excitation threshold of the nerve or neuron, optionally wherein the intensity is of 0.01 mA to 50 mA and/or 1 mV to 500 V, optionally 0.5 mA to 10 mA.
15. The device of item 13, wherein the pulse generator is configured to deliver electrical stimulation through the electrode lead/cuff at a frequency of 1 Hz to less than 4 kHz, or 1 Hz to 1.3 kHz, or 1 Hz to 1.5 kHz, optionally from 100 Hz to 1 kHz, optionally from 100 Hz to 500 Hz, for a period of from 5 seconds to 14 days, or 1 minute to 14 days, optionally from 2 minutes to 2 hours, optionally from 1 minute to 7 days, optionally from 1 minute to 5 days, optionally from 2 minutes to 5 minutes, optionally from 5 seconds to 90 seconds, wherein the electrical stimulation comprises biphasic, charge-balanced electrical pulses.
16. The device of item 13, wherein the controller is programmed or configured to, once block of nerve conduction or neuron excitation is achieved, instruct the pulse generator to stop application of the electrical stimulation for a period of at least 10 seconds, optionally at least 1 minute, optionally at least 5 minutes, 10 minutes, 15 minutes, 30 minutes, or 180 minutes, wherein the block of nerve conduction or neuron excitation is maintained during the period and, after the period has concluded, resume electrical stimulation of the nerve or neuron at the same or different intensity and/or at the same or different frequency to continue or prolong the block of nerve conduction or neuron excitation.
17. The device of item 13, wherein the controller is programmed or configured to, once block of nerve conduction or neuron excitation is achieved, instruct the pulse generator to change the intensity and/or frequency of the electrical stimulation, optionally by reducing the intensity of the electrical stimulation or increasing the frequency of the electrical stimulation.
18. A device comprising:
   a controller;
   a pulse generator in communication with the controller; and
   one or more skin surface electrodes or electromagnetic coils in electrical communication with the pulse generator, wherein
   the pulse generator and one or more skin surface electrodes or electromagnetic coils are configured to apply an electrical stimulation to a nerve or neuron, wherein the electrical stimulation is of an intensity that is equal to or greater than an excitation threshold of the nerve or neuron for a length of time sufficient to produce a block of nerve conduction or neuron excitation.
19. The device of item 18, wherein the pulse generator is configured to deliver electrical stimulation through the electrode or electromagnetic coil at an intensity that is at least five times the excitation threshold of the nerve or neuron, optionally wherein the intensity is of 0.01 mA to 50 mA and/or 1 mV to 500 V, optionally 0.5 mA to 10 mA.
20. The device of item 18, wherein the pulse generator is configured to deliver electrical stimulation through the electrode or electromagnetic coil at a frequency of 1 Hz to less than 4 kHz, or 1 Hz to 1.3 kHz, or 1 Hz to 1.5 kHz, optionally from 100 Hz to 1 kHz, optionally from 100 Hz to 500 Hz, for a period of from 5 seconds to 14 days, optionally from 2 minutes to 2 hours, optionally from 1 minute to 7 days, optionally from 1 minute to 5 days, optionally from 2 minutes to 5 minutes, optionally from 5 seconds to 90 seconds, wherein the electrical stimulation comprises biphasic, charge-balanced electrical pulses.
21. The device of item 18, wherein the controller is programmed or configured to, once block of nerve conduction or neuron excitation is achieved, instruct the pulse generator to stop application of the electrical stimulation for a period of at least 10 seconds, optionally 1 minute, optionally at least 5 minutes, 10 minutes, 15 minutes, 30 minutes, or 180 minutes, wherein the block of nerve conduction or neuron excitation is maintained during the period and, after the period has concluded, resume electrical stimulation of the nerve or neuron at the same or different intensity and/or at the same or different frequency to continue or prolong the block of nerve conduction or neuron excitation.
22. The device of item 18, wherein the controller is programmed or configured to, once block of nerve conduction or neuron excitation is achieved, instruct the pulse generator to change the intensity and/or frequency of the electrical stimulation, optionally by reducing the intensity of the electrical stimulation or increasing the frequency of the electrical stimulation.
23. A method of controlling micturition or defecation in a patient comprising:
   applying a first electrical stimulation to the left pudendal nerve or branches thereof and a second electrical stimulation to the right pudendal nerve or branches thereof of a patient, wherein the first and second electrical stimulation is of an intensity that is equal to or greater than an excitation threshold of the pudendal nerve for a length of time sufficient to produce a conduction block of both pudendal nerves either during or after ending the first and/or second electrical stimulation, thereby inhibiting contraction of the external urethral sphincter (EUS) and/or external anal sphincter (EAS),
   and either during or immediately after ending the first and/or second electrical stimulation when the EUS/EAS is relaxed, applying a third electrical stimulation to the left or right pudendal nerve at a location central to the blocked pudendal nerve site to induce bladder or colon/rectal contraction,
   or either during or immediately after ending the first and/or second electrical stimulation when the EUS/EAS is relaxed, applying a third electrical stimulation to a sacral spinal root (S1, S2, S3, or S4) to induce bladder or colon/rectal contraction,
   or either during or immediately after ending the first and/or second electrical stimulation when the EUS/EAS is relaxed, applying a third electrical stimulation to the spinal cord by epidural electrodes, or skin surface electrodes or electromagnetic coils to induce bladder or colon/rectal contraction,
   or either during or immediately after ending the first and/or second electrical stimulation when the EUS/EAS is relaxed, applying a third electrical stimulation to pelvic nerve to induce bladder or colon/rectal contraction,
   or either during or immediately after ending the first and/or second electrical stimulation and during the EUS/EAS relaxation, manually applying pressure to the abdominal area to produce bladder or colon/rectal pressure.
24. The method of item 23, wherein the first and second electrical stimulation for pudendal nerve block is delivered at an intensity that is at least five times the excitation threshold of the pudendal nerve, optionally wherein the intensity is of 0.01 mA to 50 mA and/or 1 mV to 500 V, optionally 0.5 mA to 10 mA.
25. The method of item 23, wherein the first and second electrical stimulation for pudendal nerve block is delivered at a frequency of 1 Hz to less than 4 kHz, or 1 Hz to 1.3 Hz, or 1 Hz to 1.5 kHz, optionally from 100 Hz to 1 kHz, optionally from 100 Hz to 500 Hz.
26. The method of item 23, wherein the first and second electrical stimulation for pudendal nerve block is delivered for a period of from 5 seconds to 60 minutes, 1 minute to 60 minutes, optionally from 10 seconds to 90 seconds, optionally from 30 seconds to 60 seconds, optionally from 1 minute to 3 minutes, optionally from 2 minutes to 20 minutes, optionally from 30 minutes to 60 minutes.
27. The method of item 23, wherein the first and second electrical stimulation results in the block of pudendal nerve conduction for at least 10 seconds following cessation of the electrical stimulation.
28. The method of item 23, wherein the first and second electrical stimulation comprises biphasic electrical pulses.
29. The method of item 28, wherein the biphasic pulses are symmetric between the positive and negative phases of the biphasic pulse.
30. The method of item 28, wherein the biphasic pulses are asymmetric between the positive and negative phases of the biphasic pulse.
31. The method of item 23, wherein the first, second, and third electrical stimulation comprises biphasic electrical pulses that are charge-balanced.
32. The method of item 23, further comprising, once block of pudendal nerve conduction is achieved, stopping application of the first and/or second electrical stimulation for a period of at least 10 seconds, optionally at least 1 minute, optionally at least 5 minutes, 10 minutes, 15 minutes, 30 minutes, or 180 minutes, wherein the block of pudendal nerve conduction is maintained during the period and, after the period has concluded, resuming the first and/or second electrical stimulation of the pudendal nerve or branch thereof at the same or different intensity and/or at the same or different frequency to continue or prolong the block of pudendal nerve conduction.
33. The method of item 23, further comprising, once block of pudendal nerve conduction or excitation is achieved, maintaining the block by changing the intensity and/or frequency of the first and/or second electrical stimulation, optionally by reducing the intensity of the electrical stimulation or increasing the frequency of the electrical stimulation.
34. The method of item 23, wherein the first and second stimulation comprises biphasic pulses at an intensity of 6-9 mA and a frequency of between 100 Hz and 1 kHz, applied for from 10-60 seconds.
35. The method of item 23, wherein the third electrical stimulation for inducing bladder or colon/rectal contraction and applied to the pudendal nerve, the sacral (S1, S2, S3, or S4) spinal root, spinal cord, or the pelvic nerve has a frequency between 1 Hz to 100 Hz, optionally between 15 Hz and 50 Hz, or optionally 1 Hz to 3 Hz.
36. The method of item 23, wherein the third electrical stimulation is delivered at an intensity above the excitation threshold of the nerve stimulated and strong enough to induce bladder or colon/rectal contraction causing bladder or colon/rectal pressure to increase more than 20 cm H₂O, optionally wherein the stimulation intensity is of 0.01 mA to 20 mA and/or 1 mV to 20 V, optionally 0.1 mA to 10 mA.
37. The method of item 35, wherein the third stimulation is delivered for a period longer than the period of bladder or colon/rectal contraction, optionally for a period of from 10 seconds to 60 minutes, optionally from 30 seconds to 60 seconds, or from 30 seconds to 2 minutes, or from 30 seconds to 3 minutes, or from 30 minutes to 60 minutes.
38. The method of item 35, further comprising, once the bladder or colon/rectal contraction has ended, stopping application of the third electrical stimulation for a period of at least 10 seconds, optionally at least 1 minute, optionally at least 5 minutes, 10 minutes, 15 minutes, or 30 minutes, after the period has concluded, resuming the third stimulation at the same or different intensity and/or at the same or different frequency to induce bladder or colon contraction again while the pudendal nerves are blocked and the EUS/EAS is relaxed.
39. A method of controlling micturition or defecation in a patient comprising:
   applying first electrical stimulation to either left or right pudendal nerve or branches thereof of a subject, wherein the electrical stimulation is of an intensity that is equal to or greater than an excitation threshold of the pudendal nerve for a length of time sufficient to produce a block of pudendal nerve conduction either during or after ending the electrical stimulation, thereby inhibiting contraction of the external urethral sphincter (EUS) and/or external anal sphincter (EAS),
   and either during or immediately after ending the first electrical stimulation when the EUS/EAS is relaxed, applying a second electrical stimulation to the same side of the blocked pudendal nerve at a location central to the blocked pudendal nerve site to induce bladder or colon/rectal contraction,
   or either during or immediately after ending the first electrical stimulation when the EUS/EAS is relaxed, applying a second electrical stimulation to a sacral spinal root (S1, S2, S3, or S4) to induce bladder or colon/rectal contraction,
   or either during or immediately after ending the first electrical stimulation when the EUS/EAS is relaxed, applying a second electrical stimulation to the spinal cord by epidural electrodes, or skin surface electrodes or electromagnetic coils to induce bladder or colon/rectal contraction,
   or either during or immediately after ending the first electrical stimulation when the EUS/EAS is relaxed, applying a second electrical stimulation to pelvic nerve to induce bladder or colon/rectal contraction,
   or either during or immediately after ending the electrical stimulation when the EUS/EAS is relaxed, manually applying pressure to the abdominal area to produce bladder or colon/rectal pressure.
40. The method of item 39, wherein the first electrical stimulation for pudendal nerve block is delivered at an intensity that is at least five times the excitation threshold of the pudendal nerve, optionally wherein the intensity is of 0.01 mA to 50 mA and/or 1 mV to 500 V, optionally 0.5 mA to 10 mA.
41. The method of item 39, wherein the first electrical stimulation for pudendal nerve block is delivered at a frequency of 1 Hz to less than 4 kHz, or 1 Hz to 1.3 Hz, or 1 Hz to 1.5 kHz, optionally from 100 Hz to 1 kHz, optionally from 100 Hz to 500 Hz.
42. The method of item 39, wherein the first electrical stimulation for pudendal nerve block is delivered for a period of from 5 seconds to 60 minutes, 1 minute to 60 minutes, optionally from 10 seconds to 90 seconds, optionally from 30 seconds to 60 seconds, optionally from 1 minute to 3 minutes, optionally from 2 minutes to 20 minutes, optionally from 30 minutes to 60 minutes.
43. The method of any of items 39-42, wherein the first electrical stimulation results in the block of pudendal nerve conduction for at least 10 seconds following cessation of the electrical stimulation.
44. The method of item 39, wherein the first electrical stimulation comprises biphasic electrical pulses.
45. The method of item 44, wherein the biphasic pulses are symmetric between the positive and negative phases of the biphasic pulse.
46. The method of item 44, wherein the biphasic pulses are asymmetric between the positive and negative phases of the biphasic pulse.
47. The method of item 39, wherein the first and second electrical stimulation comprises biphasic electrical pulses that are charge-balanced.
48. The method of item 39, further comprising, once block of pudendal nerve conduction is achieved, stopping application of the first electrical stimulation for a period of at least 10 seconds, optionally at least 1 minute, optionally at least 5 minutes, 10 minutes, 15 minutes, 30 minutes, or 180 minutes, wherein the block of pudendal nerve conduction is maintained during the period and, after the period has concluded, resuming the first electrical stimulation of the pudendal nerve or branch thereof at the same or different intensity and/or at the same or different frequency to continue or prolong the block of pudendal nerve conduction.
49. The method of item 39, further comprising, once block of pudendal nerve conduction or excitation is achieved, maintaining the block by changing the intensity and/or frequency of the first electrical stimulation, optionally by reducing the intensity of the electrical stimulation or increasing the frequency of the electrical stimulation.
50. The method of item 39, wherein the first stimulation comprises biphasic pulses at an intensity of 6-9 mA and a frequency of between 100 Hz and 1 kHz, applied for from 10-60 seconds.
51. The method of item 39, wherein the second electrical stimulation for inducing bladder or colon/rectal contraction and applied to the pudendal nerve, the sacral (S1, S2, S3, or S4) spinal root, spinal cord, or the pelvic nerve has a frequency between 1 Hz to 100 Hz, optionally between 15 Hz and 50 Hz, or optionally between 1 Hz and 3 Hz.
52. The method of item 51, wherein the second electrical stimulation is delivered at an intensity above the excitation threshold of the nerve stimulated and strong enough to induce bladder or colon/rectal contraction causing bladder or colon/rectal pressure to increase more than 20 cm H₂O, optionally wherein the stimulation intensity is of 0.01 mA to 20 mA and/or 1 mV to 20 V, optionally 0.1 mA to 10 mA.
53. The method of item 51, wherein the second stimulation is delivered for a period longer than the period of bladder or colon/rectal contraction, optionally for a period of from 10 seconds to 60 minutes, optionally from 30 seconds to 60 seconds, or from 30 seconds to 2 minutes, or from 30 seconds to 3 minutes, or from 30 minutes to 60 minutes.
54. The method of item 51, further comprising, once the bladder or colon/rectal contraction has ended, stopping application of the second electrical stimulation for a period of at least 10 seconds, optionally at least 1 minute, optionally at least 5 minutes, 10 minutes, 15 minutes, or 30 minutes, after the period has concluded, resuming the second stimulation at the same or different intensity and/or at the same or different frequency to induce bladder or colon/rectal contraction again while the pudendal nerves are blocked and the EUS/EAS is relaxed.
55. A device of controlling micturition or defecation in a patient comprising:
   a controller;
   one or two pulse generators in communication with the controller; and
   one or more lead and/or cuff electrodes, optionally two electrodes, configured to be placed near or in contact with the left and/or right pudendal nerves, the electrodes in electrical communication with the first pulse generator, wherein
   the device is configured to apply an electrical stimulation to the pudendal nerve, wherein the electrical stimulation is of an intensity that is equal to or greater than an excitation threshold of the pudendal nerve for a length of time sufficient to produce a block of pudendal nerve conduction either during or after ending the electrical stimulation, thereby inhibiting contraction of the external urethral sphincter (EUS) and/or the external anal sphincter (EAS); and
   another electrode, configured to be placed near or in contact with the blocked pudendal nerve at a site central to the blocked site, or be placed near or in contact with the sacral (S1, S2, S3, or S4) spinal root, spinal cord, or pelvic nerve, the electrode in electrical communication with the first pulse generator, wherein
   the device is configured to apply electrical stimulation using the electrode either during or immediately after the ending of pudendal nerve blocking stimulation at an intensity that is greater than an excitation threshold of the pudendal nerve, sacral (S1, S2, S3, or S4) spinal root, spinal cord, or pelvic nerve to induce bladder or colon/rectal contraction; or
   skin surface electrodes or electromagnetic coils, configured to be placed on the skin surface along the patient's spine and in electrical communication with the second pulse generator, and wherein
   the device is configured to apply electrical stimulation using the skin surface electrodes or electromagnetic coils either during or immediately after the ending of pudendal nerve blocking stimulation at an intensity that is greater than an excitation threshold of the spinal cord to induce bladder or colon/rectal contraction.
56. The device of item 55, wherein the electrical stimulation for pudendal nerve block is delivered at an intensity that is at least five times the excitation threshold of the pudendal nerve, optionally wherein the intensity is of 0.01 mA to 50 mA and/or 1 mV to 500 V, optionally 0.5 mA to 10 mA.
57. The device of item 55, wherein the electrical stimulation for pudendal nerve block is delivered at a frequency of 1 Hz to less than 4 kHz, or 1 Hz to 1.3 Hz, or 1 Hz to 1.5 kHz, optionally from 100 Hz to 1 kHz, optionally from 100 Hz to 500 Hz.
58. The device of item 55, wherein the electrical stimulation for pudendal nerve block is delivered for a period of from 5 seconds to 60 minutes, 1 minute to 60 minutes, optionally from 10 seconds to 90 seconds, optionally from 30 seconds to 60 seconds, optionally from 1 minute to 5 minutes, optionally from 2 minutes to 20 minutes, optionally from 30 minutes to 60 minutes.
59. The device of item 55, wherein the electrical stimulation results in the block of pudendal nerve conduction for at least 10 seconds following cessation of the electrical stimulation.
60. The device of item 55, wherein the electrical stimulation for pudendal nerve block comprises biphasic electrical pulses.
61. The device of item 60, wherein the biphasic pulses are symmetric between the positive and negative phases of the biphasic pulse.
62. The device of item 60, wherein the biphasic pulses are asymmetric between the positive and negative phases of the biphasic pulse.
63. The device of item 55, wherein the electrical stimulation comprises biphasic electrical pulses that are charge-balanced.
64. The device of item 55, further comprising, once block of pudendal nerve conduction is achieved, stopping application of the pudendal nerve blocking electrical stimulation for a period of at least 10 seconds, optionally at least 1 minute, optionally at least 5 minutes, 10 minutes, 15 minutes, 30 minutes, or 180 minutes, wherein the block of pudendal nerve conduction is maintained during the period and, after the period has concluded, resuming the electrical stimulation of the pudendal nerve or branch thereof at the same or different intensity and/or at the same or different frequency to continue or prolong the block of pudendal nerve conduction.
65. The device of item 55, further comprising, once block of pudendal nerve conduction is achieved, maintaining the block by changing the intensity and/or frequency of the pudendal nerve blocking electrical stimulation, optionally by reducing the intensity of the electrical stimulation or increasing the frequency of the electrical stimulation.
66. The device of item 55, wherein the electrical stimulation for inducing bladder or colon/rectal contraction and applied to the pudendal nerve, the sacral (S1, S2, S3, or S4) spinal root, spinal cord, or the pelvic nerve has a frequency between 1 Hz to 100 Hz, optionally between 15 Hz and 50 Hz, or optionally between 1 Hz to 3 Hz.
67. The device of item 66, wherein the stimulation is delivered at an intensity above the excitation threshold of the nerve stimulated and strong enough to induce bladder contraction causing bladder or colon/rectal pressure to increase more than 20 cm H₂O, optionally wherein the intensity is of 0.01 mA to 20 mA and/or 1 mV to 20 V, optionally 0.1 mA to 10 mA.
68. The device of item 66, wherein the stimulation is delivered for a period longer than the period of bladder or colon/rectal contraction, optionally for a period of from 10 seconds to 60 minutes, optionally from 30 seconds to 60 seconds, or from 30 seconds to 2 minutes, or from 30 seconds to 3 minutes, or from 30 minutes to 60 minutes.
69. The device of item 66, further comprising, once the bladder or colon/rectal contraction has ended, stopping application of the electrical stimulation for a period of at least 10 seconds, optionally at least 1 minute, optionally at least 5 minutes, 10 minutes, 15 minutes, 30 minutes, or 180 minutes, after the period has concluded, resuming the stimulation at the same or different intensity and/or at the same or different frequency to induce bladder or colon/rectal contraction again while the pudendal nerves are blocked and the EUS/EAS is relaxed.
70. A device of controlling micturition or defecation in a patient comprising:
   a controller;
   a pulse generator in communication with the controller; and
   one or more lead and/or cuff electrodes, optionally two electrodes, configured to be placed near or in contact with the left and/or right pudendal nerves, the electrodes in electrical communication with the pulse generator, wherein
   the device is configured to apply an electrical stimulation to the pudendal nerve, wherein the electrical stimulation is of an intensity that is equal to or greater than an excitation threshold of the pudendal nerve for a length of time sufficient to produce a block of pudendal nerve conduction either during or after ending the electrical stimulation, thereby inhibiting contraction of the external urethral sphincter (EUS) and/or the external anal sphincter (EAS).
71. The device of item 70, wherein the electrical stimulation for pudendal nerve block is delivered at an intensity that is at least five times the excitation threshold of the pudendal nerve, optionally wherein the intensity is of 0.01 mA to 50 mA and/or 1 mV to 500 V, optionally 0.5 mA to 10 mA.
72. The device of item 70, wherein the electrical stimulation for pudendal nerve block is delivered at a frequency of 1 Hz to less than 4 kHz, or 1 Hz to 1.3 Hz, or 1 Hz to 1.5 kHz, optionally from 100 Hz to 1 kHz, optionally from 100 Hz to 500 Hz.
73. The device of item 70, wherein the electrical stimulation for pudendal nerve block is delivered for a period of from 5 seconds to 60 minutes, 1 minute to 60 minutes, optionally from 10 seconds to 90 seconds, optionally from 30 seconds to 60 seconds, optionally from 1 minute to 5 minutes, optionally from 2 minutes to 20 minutes, optionally from 30 minutes to 60 minutes.
74. The device of item 70, wherein the electrical stimulation results in the block of pudendal nerve conduction for at least 10 seconds following cessation of the electrical stimulation.
75. The device of item 70, wherein the electrical stimulation comprises biphasic electrical pulses.
76. The method of item 75, wherein the biphasic pulses are symmetric between the positive and negative phases of the biphasic pulse.
77. The method of item 75, wherein the biphasic pulses are asymmetric between the positive and negative phases of the biphasic pulse.
78. The method of item 70, wherein the electrical stimulation comprises biphasic electrical pulses that are charge-balanced.
79. The device of item 70, further comprising, once block of pudendal nerve conduction is achieved, stopping application of the electrical stimulation for a period of at least 10 seconds, optionally at least 1 minute, optionally at least 5 minutes, 10 minutes, 15 minutes, 30 minutes, or 180 minutes, wherein the block of pudendal nerve conduction is maintained during the period and, after the period has concluded, resuming electrical stimulation of the pudendal nerve or branch thereof at the same or different intensity and/or at the same or different frequency to continue or prolong the block of pudendal nerve conduction.
80. The device of item 70, further comprising, once block of pudendal nerve conduction is achieved, maintaining the block by changing the intensity and/or frequency of the electrical stimulation, optionally by reducing the intensity of the electrical stimulation or increasing the frequency of the electrical stimulation.

## Claims

1. A device for blocking nerve conduction or neuron excitation in a patient comprising:
a controller;
a pulse generator in communication with the controller; and either:
an electrode lead or cuff configured to be placed near or in contact with a nerve or neuron, the electrode lead/cuff being in electrical communication with the pulse generator; or
one or more skin surface electrodes or electromagnetic coils in electrical communication with the pulse generator and configured to placed in proximity to the nerve or neuron,
wherein,
the device is configured to apply an electrical stimulation to the nerve or neuron, wherein the electrical stimulation is of an intensity that is equal to or greater than an excitation threshold of the nerve or neuron, for a length of time sufficient to produce a block of nerve conduction or neuron excitation either during or after ending the electrical stimulation, wherein the electrical stimulation for nerve or neuron block is delivered at a frequency of 1 Hz to less than 4 kHz

2. The device of claim 1, wherein the pulse generator is configured to deliver electrical stimulation through the electrode lead/cuff or electrode/coil at an intensity that is at least five times the excitation threshold of the nerve or neuron.

3. The device of claim 1, wherein the pulse generator is configured to deliver electrical stimulation through the electrode lead/cuff or electrode/coil at an intensity of 0.01 mA to 50 mA and/or 1 mV to 500 V.

4. The device of claim 1, wherein the pulse generator is configured to deliver electrical stimulation through the electrode lead/cuff or electrode/coil at an intensity of 0.5 mA to 10 mA.

5. The device of claim 1, wherein the pulse generator is configured to deliver electrical stimulation through the electrode lead/cuff or electrode/coil at a frequency of from 100 Hz to 1 kHz.

6. The device of claim 1, wherein the pulse generator is configured to deliver electrical stimulation through the electrode lead/cuff or electrode/coil at a frequency of 100 Hz to 500 Hz.

7. The device of claim 5 or claim 6, wherein the pulse generator is configured to deliver electrical stimulation through the electrode lead/cuff or electrode/coil for a period of from 5 seconds to 14 days.

8. The device of claim 1, wherein the electrical stimulation for pudendal nerve block comprises biphasic electrical pulses.

9. The device of claim 8, wherein the biphasic pulses are symmetric between the positive and negative phases of the biphasic pulse.

10. The device of claim 8, wherein the biphasic pulses are asymmetric between the positive and negative phases of the biphasic pulse.

11. The device of claim 1, wherein the electrical stimulation comprises biphasic electrical pulses that are charge-balanced.

12. The device of claim 1, wherein the controller is programmed or configured to, once block of nerve conduction or neuron excitation is achieved, instruct the pulse generator to stop application of the electrical stimulation for a period of at least 10 seconds, wherein the block of nerve conduction or neuron excitation is maintained during the period and, after the period has concluded, resumes electrical stimulation of the nerve or neuron at the same or different intensity and/or at the same or different frequency to continue or prolong the block of nerve conduction or neuron excitation.

13. The device of claim 11, wherein the controller is programmed or configured to, once block of nerve conduction or neuron excitation is achieved, instruct the pulse generator to change the intensity and/or frequency of the electrical stimulation.

14. The device of claim 13, wherein the controller is programmed to instruct the pulse generator to reduce the intensity of the electrical stimulation.

15. The device of claim 13, wherein the controller is programmed to instruct the pulse generator to increase the frequency of the electrical stimulation.
